# EUROPÄISCHE PATENTANMELDUNG

(11) **EP 3 770 242 A1**
(43) Veröffentlichungstag der Anmeldung: **27.01.2021**
(21) Anmeldenummer: 19187523.6
(22) Anmeldetag: 22.07.2019
(51) Int. Cl.: C11D 17/00, C11D 11/00, C11D 3/386, C11D 17/04, C11D 17/08

(54) **REINIGUNGSMITTEL MIT ENZYM**

(71) Anmelder: Henkel AG & Co. KGaA, 40589 Düsseldorf (DE)
(72) Erfinder: Weber, Thomas, 35096 Weimar (Lahn) (DE); Bode, Nicole, 40627 Düsseldorf (DE); Doering, Thomas, 41540 Dormagen (DE); Dorra, Klaus, 40591 Düsseldorf (DE); Strauss, Britta, 42697 Solingen (DE); Mussmann, Nina, 47877 Willich (DE); Degering, Christian, 40699 Erkrath (DE); Bastigkeit, Thorsten, 42279 Wuppertal (DE); Wieland, Susanne, 41541 Zons/Dormagen (DE)

(57) **Zusammenfassung**

Die Erfindung betrifft Reinigungsmittel, bevorzugt Geschirrspülmittel, insbesondere das maschinelle Geschirrspülmittel, umfassend mindestens eine wasserarme, bevorzugt im wesentlichen wasserfreie gelförmige Phase, welche mindestens ein Enzym, bevorzugt Enzymgranulat, enthält.

SEQUENCE LISTING

<110>  Henkel AG & Co. KGaA

<120>  Reinigungsmittel mit Enzym

<130>  2019P00253EP

<160>  7

<170>  PatentIn version 3.5

<210>  1
<211>  269
<212>  PRT
<213>  Bacillus gibsonii

<400>  1

Gln Gln Thr Val Pro Trp Gly Ile Thr Arg Val Gln Ala Pro Thr Val
1               5                   10                  15

His Asn Arg Gly Ile Thr Gly Ser Gly Val Lys Val Ala Ile Leu Asp
            20                  25                  30

Thr Gly Ile Ala Gln His Ser Asp Leu Thr Ile Arg Gly Gly Ala Ser
        35                  40                  45

Phe Val Pro Gly Glu Ser Thr Thr Ala Asp Leu Asn Gly His Gly Thr
    50                  55                  60

His Val Ala Gly Thr Val Ala Ala Leu Asn Asn Ser Ile Gly Val Ile
65                  70                  75                  80

Gly Val Ala Pro Ser Ala Asp Leu Tyr Ala Val Lys Val Leu Gly Ala
                85                  90                  95

Asn Gly Arg Gly Ser Val Ser Gly Ile Ala Gln Gly Leu Glu Trp Ala
            100                 105                 110

Ala Thr Asn Asn Met His Ile Ala Asn Met Ser Leu Gly Ser Asp Ala
        115                 120                 125

Pro Ser Thr Thr Leu Glu Arg Ala Val Asn Tyr Ala Thr Ser Arg Gly
    130                 135                 140

Val Leu Val Ile Ala Ala Thr Gly Asn Asn Gly Thr Gly Ser Ile Gly
145                 150                 155                 160

Tyr Pro Ala Arg Tyr Ala Asn Ala Met Ala Val Gly Ala Thr Asp Gln
                165                 170                 175

Asn Asn Arg Arg Ala Ser Phe Ser Gln Tyr Gly Thr Gly Ile Asp Ile
            180                 185                 190

Val Ala Pro Gly Val Gly Ile Gln Ser Thr Tyr Leu Asn Asn Ser Tyr
        195                 200                 205

Ala Ser Met Pro Gly Thr Ser Met Ala Thr Pro His Val Ala Gly Val
    210                 215                 220

Ala Ala Leu Val Lys Gln Lys Asn Pro Ser Trp Asn Ala Thr Gln Ile
225                 230                 235                 240

Arg Asn His Leu Lys Asn Thr Ala Thr Asn Leu Gly Asn Ser Ser Gln
                245                 250                 255

Phe Gly Ser Gly Leu Val Asn Ala Asp Ala Ala Thr Arg
            260                 265

<210>  2
<211>  269
<212>  PRT
<213>  Bacillus lentus

<400>  2

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>  3
<211>  269
<212>  PRT
<213>  Bacillus alkalophilus

<400>  3

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Asn Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>  4
<211>  269
<212>  PRT
<213>  Bacillus lentus

<400>  4

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Asp Gly Arg Gly Ala Ile Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Ser Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>  5
<211>  485
<212>  PRT

<213>  Bacillus sp.

<400>  5

His His Asn Gly Thr Asn Gly Thr Met Met Gln Tyr Phe Glu Trp Tyr
1               5                   10                  15

Leu Pro Asn Asp Gly Asn His Trp Asn Arg Leu Asn Ser Asp Ala Ser
            20                  25                  30

Asn Leu Lys Ser Lys Gly Ile Thr Ala Val Trp Ile Pro Pro Ala Trp
        35                  40                  45

Lys Gly Ala Ser Gln Asn Asp Val Gly Tyr Gly Ala Tyr Asp Leu Tyr
    50                  55                  60

Asp Leu Gly Glu Phe Asn Gln Lys Gly Thr Val Arg Thr Lys Tyr Gly
65                  70                  75                  80

Thr Arg Ser Gln Leu Gln Ala Ala Val Thr Ser Leu Lys Asn Asn Gly
                85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Thr Gly Glu Tyr Thr Ile Glu Ala Trp Thr Arg Phe Asp
    130                 135                 140

Phe Pro Gly Arg Gly Asn Thr His Ser Ser Phe Lys Trp Arg Trp Tyr
145                 150                 155                 160

His Phe Asp Gly Val Asp Trp Asp Gln Ser Arg Arg Leu Asn Asn Arg
                165                 170                 175

Ile Tyr Lys Phe Arg Gly His Gly Lys Ala Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
            245                 250                 255

Thr Gly Lys Asn Met Phe Ala Val Ala Glu Phe Trp Lys Asn Asp Leu
        260                 265                 270

Gly Ala Ile Glu Asn Tyr Leu Gln Lys Thr Asn Trp Asn His Ser Val
    275                 280                 285

Phe Asp Val Pro Leu His Tyr Asn Leu Tyr Asn Ala Ser Lys Ser Gly
    290                 295                 300

Gly Asn Tyr Asp Met Arg Asn Ile Phe Asn Gly Thr Val Val Gln Arg
305                 310                 315                 320

His Pro Ser His Ala Val Thr Phe Val Asp Asn His Asp Ser Gln Pro
            325                 330                 335

Glu Glu Ala Leu Glu Ser Phe Val Glu Glu Trp Phe Lys Pro Leu Ala
        340                 345                 350

Tyr Ala Leu Thr Leu Thr Arg Glu Gln Gly Tyr Pro Ser Val Phe Tyr
    355                 360                 365

Gly Asp Tyr Tyr Gly Ile Pro Thr His Gly Val Pro Ala Met Arg Ser
    370                 375                 380

Lys Ile Asp Pro Ile Leu Glu Ala Arg Gln Lys Tyr Ala Tyr Gly Lys
385                 390                 395                 400

Gln Asn Asp Tyr Leu Asp His His Asn Ile Ile Gly Trp Thr Arg Glu
            405                 410                 415

Gly Asn Thr Ala His Pro Asn Ser Gly Leu Ala Thr Ile Met Ser Asp
        420                 425                 430

Gly Ala Gly Gly Ser Lys Trp Met Phe Val Gly Arg Asn Lys Ala Gly
    435                 440                 445

Gln Val Trp Ser Asp Ile Thr Gly Asn Arg Thr Gly Thr Val Thr Ile
    450                 455                 460

Asn Ala Asp Gly Trp Gly Asn Phe Ser Val Asn Gly Gly Ser Val Ser
465                 470                 475                 480

Ile Trp Val Asn Lys
            485

<210>  6
<211>  485
<212>  PRT
<213>  Bacillus sp.

<400>  6

His His Asn Gly Thr Asn Gly Thr Met Met Gln Tyr Phe Glu Trp Tyr
1               5                   10                  15

Leu Pro Asn Asp Gly Asn His Trp Asn Arg Leu Arg Ser Asp Ala Ser
            20                  25                  30

Asn Leu Lys Asp Lys Gly Ile Ser Ala Val Trp Ile Pro Pro Ala Trp
        35                  40                  45

Lys Gly Ala Ser Gln Asn Asp Val Gly Tyr Gly Ala Tyr Asp Leu Tyr
    50                  55                  60

Asp Leu Gly Glu Phe Asn Gln Lys Gly Thr Ile Arg Thr Lys Tyr Gly
65                  70                  75                  80

Thr Arg Asn Gln Leu Gln Ala Ala Val Asn Ala Leu Lys Ser Asn Gly
                85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Ser Gly Glu Tyr Thr Ile Glu Ala Trp Thr Lys Phe Asp
    130                 135                 140

Phe Pro Gly Arg Gly Asn Thr His Ser Asn Phe Lys Trp Arg Trp Tyr
145                 150                 155                 160

His Phe Asp Gly Val Asp Trp Asp Gln Ser Arg Lys Leu Asn Asn Arg
                165                 170                 175

Ile Tyr Lys Phe Arg Gly Asp Gly Lys Gly Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
                245                 250                 255

Thr Gly Lys Asn Met Phe Ala Val Ala Glu Phe Trp Lys Asn Asp Leu
            260                 265                 270

Gly Ala Ile Glu Asn Tyr Leu Asn Lys Thr Asn Trp Asn His Ser Val
        275                 280                 285

Phe Asp Val Pro Leu His Tyr Asn Leu Tyr Asn Ala Ser Lys Ser Gly
    290                 295                 300

Gly Asn Tyr Asp Met Arg Gln Ile Phe Asn Gly Thr Val Val Gln Arg
305                 310                 315                 320

His Pro Met His Ala Val Thr Phe Val Asp Asn His Asp Ser Gln Pro
                325                 330                 335

Glu Glu Ala Leu Glu Ser Phe Val Glu Glu Trp Phe Lys Pro Leu Ala
            340                 345                 350

Tyr Ala Leu Thr Leu Thr Arg Glu Gln Gly Tyr Pro Ser Val Phe Tyr
        355                 360                 365

Gly Asp Tyr Tyr Gly Ile Pro Thr His Gly Val Pro Ala Met Lys Ser
    370                 375                 380

Lys Ile Asp Pro Ile Leu Glu Ala Arg Gln Lys Tyr Ala Tyr Gly Arg
385                 390                 395                 400

Gln Asn Asp Tyr Leu Asp His His Asn Ile Ile Gly Trp Thr Arg Glu
                405                 410                 415

Gly Asn Thr Ala His Pro Asn Ser Gly Leu Ala Thr Ile Met Ser Asp
            420                 425                 430

Gly Ala Gly Gly Asn Lys Trp Met Phe Val Gly Arg Asn Lys Ala Gly
        435                 440                 445

Gln Val Trp Thr Asp Ile Thr Gly Asn Arg Ala Gly Thr Val Thr Ile
    450                 455                 460

Asn Ala Asp Gly Trp Gly Asn Phe Ser Val Asn Gly Gly Ser Val Ser

465                 470                 475                 480

Ile Trp Val Asn Lys
                485

<210>  7
<211>  485
<212>  PRT
<213>  Bacillus sp.

<400>  7

His His Asn Gly Thr Asn Gly Thr Met Met Gln Tyr Phe Glu Trp His
1               5                   10                  15

Leu Pro Asn Asp Gly Asn His Trp Asn Arg Leu Arg Asp Asp Ala Ser
            20                  25                  30

Asn Leu Arg Asn Arg Gly Ile Thr Ala Ile Trp Ile Pro Pro Ala Trp
        35                  40                  45

Lys Gly Thr Ser Gln Asn Asp Val Gly Tyr Gly Ala Tyr Asp Leu Tyr
    50                  55                  60

Asp Leu Gly Glu Phe Asn Gln Lys Gly Thr Val Arg Thr Lys Tyr Gly
65                  70                  75                  80

Thr Arg Ser Gln Leu Glu Ser Ala Ile His Ala Leu Lys Asn Asn Gly
                85                  90                  95

Val Gln Val Tyr Gly Asp Val Val Met Asn His Lys Gly Gly Ala Asp
            100                 105                 110

Ala Thr Glu Asn Val Leu Ala Val Glu Val Asn Pro Asn Asn Arg Asn
        115                 120                 125

Gln Glu Ile Ser Gly Asp Tyr Thr Ile Glu Ala Trp Thr Lys Phe Asp
    130                 135                 140

Phe Pro Gly Arg Gly Asn Thr Tyr Ser Asp Phe Lys Trp Arg Trp Tyr
145                 150                 155                 160

His Phe Asp Gly Val Asp Trp Asp Gln Ser Arg Gln Phe Gln Asn Arg
                165                 170                 175

Ile Tyr Lys Phe Arg Gly Asp Gly Lys Ala Trp Asp Trp Glu Val Asp
            180                 185                 190

Ser Glu Asn Gly Asn Tyr Asp Tyr Leu Met Tyr Ala Asp Val Asp Met

        195                 200                 205

Asp His Pro Glu Val Val Asn Glu Leu Arg Arg Trp Gly Glu Trp Tyr
    210                 215                 220

Thr Asn Thr Leu Asn Leu Asp Gly Phe Arg Ile Asp Ala Val Lys His
225                 230                 235                 240

Ile Lys Tyr Ser Phe Thr Arg Asp Trp Leu Thr His Val Arg Asn Ala
                245                 250                 255

Thr Gly Lys Glu Met Phe Ala Val Ala Glu Phe Trp Lys Asn Asp Leu
            260                 265                 270

Gly Ala Leu Glu Asn Tyr Leu Asn Lys Thr Asn Trp Asn His Ser Val
        275                 280                 285

Phe Asp Val Pro Leu His Tyr Asn Leu Tyr Asn Ala Ser Asn Ser Gly
    290                 295                 300

Gly Asn Tyr Asp Met Ala Lys Leu Leu Asn Gly Thr Val Val Gln Lys
305                 310                 315                 320

His Pro Met His Ala Val Thr Phe Val Asp Asn His Asp Ser Gln Pro
                325                 330                 335

Gly Glu Ser Leu Glu Ser Phe Val Gln Glu Trp Phe Lys Pro Leu Ala
            340                 345                 350

Tyr Ala Leu Ile Leu Thr Arg Glu Gln Gly Tyr Pro Ser Val Phe Tyr
        355                 360                 365

Gly Asp Tyr Tyr Gly Ile Pro Thr His Ser Val Pro Ala Met Lys Ala
    370                 375                 380

Lys Ile Asp Pro Ile Leu Glu Ala Arg Gln Asn Phe Ala Tyr Gly Thr
385                 390                 395                 400

Gln His Asp Tyr Phe Asp His His Asn Ile Ile Gly Trp Thr Arg Glu
                405                 410                 415

Gly Asn Thr Thr His Pro Asn Ser Gly Leu Ala Thr Ile Met Ser Asp
            420                 425                 430

Gly Pro Gly Gly Glu Lys Trp Met Tyr Val Gly Gln Asn Lys Ala Gly
        435                 440                 445

Gln Val Trp His Asp Ile Thr Gly Asn Lys Pro Gly Thr Val Thr Ile
    450                 455                 460

Asn Ala Asp Gly Trp Ala Asn Phe Ser Val Asn Gly Gly Ser Val Ser
465                 470                 475                 480

Ile Trp Val Lys Arg
                485

## Beschreibung

Die Erfindung betrifft Reinigungsmittel, bevorzugt Geschirrspülmittel, insbesondere das maschinelle Geschirrspülmittel, umfassend mindestens eine wasserarme, bevorzugt im wesentlichen wasserfreie gelförmige Phase, welche mindestens ein Enzym, bevorzugt Enzymgranulat, enthält.

Wasch- oder Reinigungsmittel liegen üblicherweise in fester Form (beispielsweise als Tabletten) oder in flüssiger Form (oder auch als fließendes Gel) vor. Insbesondere flüssige Wasch- oder Reinigungsmittel erfreuen sich dabei zunehmender Beliebtheit beim Verbraucher. Vorportionierte Angebotsformen sind bei den Verbrauchern wegen der leichteren Dosierung beliebt. Dabei sind vorportionierte fließende Gele häufig problematisch, weil sie, beispielsweise wenn in Ein- oder Mehrkammerbeutel konfektioniert zu Leckagen neigen.

Aufgabe der vorliegenden Erfindung ist die Bereitstellung von Reinigungsmitteln, insbesondere Geschirrspülmitteln, bevorzugt maschinellen Geschirrspülmitteln, die sich einfach und kosteneffizient produzieren lassen, wobei gleichzeitige eine Verbesserung der Reinigungsleistung, insbesondere auf enzymsensitiven Anschmutzungen erreicht wird.

Ein erster Gegenstand der vorliegenden Erfindung betrifft somit Reinigungsmittel, umfassend eine wasserarme, bevorzugt im wesentlichen wasserfreie gelförmige Phase, welche mindestens ein Enzym enthält.

Ein Vorteil der Erfindung ist, dass die resultierenden Reinigungsmittel eine gute Stabilität, insbesondere Stabilität bei Lagerung, sowie eine verbesserte Reinigungsleistung, insbesondere gegenüber Verschmutzungen wie Creme Brûlée, Eigelb und Spaghetti, aufweisen.

Überraschenderweise wurde gefunden, dass die erfindungsgemäßen Reinigungsmittel eine besonders gute Reinigungsleistung auf enzymsensitiven Anschmutzungen aufweisen. Insbesondere bemerkenswert ist es, dass die Reinigungsmittel auch auf nicht proteasespezifischen Anschmutzungen eine verbesserte Reinigungsleistung aufweisen.

In einer bevorzugten Ausführungsform umfasst das Reinigungsmittel, bevorzugt Geschirrspülmittel, insbesondere das maschinelle Geschirrspülmittel, mindestens eine wasserarme, bevorzugt im wesentlichen wasserfreie gelförmige Phase, welche mindestens ein Enzymgranulat. Bevorzugt liegt daher das mindestens eine Enzym in Form eines Granulats vor. Insbesondere bevorzugt liegen alle Enzyme in der Gelphase als Granulate vor.

Dies hat den Vorteil, dass einerseits die Stabilität des bzw. der Enzyme sehr gut gewährleistet ist. Außerdem erfreuen sich sichtbare Partikel, z.B. von Enzymgranulaten, in transparenten Reinigungsmittelphasen bei den Verbrauchern aufgrund ihrer ansprechenden Optik einer großen Beliebtheit.

Zu den in der Gelphase enthaltenen Enzym(en) gehören insbesondere Proteasen, Amylasen, Lipasen, Hemicellulasen, Cellulasen, Perhydrolasen oder Oxidoreduktasen, sowie vorzugsweise deren Gemische. Diese Enzyme sind im Prinzip natürlichen Ursprungs; ausgehend von den natürlichen Molekülen stehen für den Einsatz in Reinigungsmitteln verbesserte Varianten zur Verfügung, die entsprechend bevorzugt eingesetzt werden. Erfindungsgemäße Reinigungsmittel enthalten Enzyme vorzugsweise in Gesamtmengen von 1 x 10⁻⁶ Gew.-% bis 5 Gew.-% bezogen auf aktives Protein. Die Proteinkonzentration kann mit Hilfe bekannter Methoden, zum Beispiel dem BCA-Verfahren oder dem Biuret-Verfahren bestimmt werden.

Gemäß einer bevorzugten Ausführungsform beträgt der Gewichtsanteil aller Enzyme (bezogen auf die Menge an aktivem Enzymprotein), in der mindestens einen gelförmigen Phase, bezogen auf das Gesamtgewicht der gelförmigen Phase(n), 0,001 bis 10,0 Gew.-%, bevorzugt 0,01 bis 7,5 Gew.-%, weiter bevorzugt 0,05 bis 6,0 Gew.-% und noch weiter bevorzugt 0,1 bis 5,0 Gew.-%.

Unter den Proteasen sind solche vom Subtilisin-Typ bevorzugt. Beispiele hierfür sind die Subtilisine BPN' und Carlsberg sowie deren weiterentwickelte Formen, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7.

Gemäß einer besonders bevorzugten Ausführungsform umfasst die wenigstens eine Gelphase, mindestens ein Enzym, bevorzugt Enzymgranulat, ausgewählt aus der Gruppe der Proteasen, bevorzugt ausgewählt aus der Gruppe der Subtilisine (bzw. ein Subtilisin-haltiges Enzym), wobei die bevorzugt einzusetzende(n) Protease(n) ausgewählt ist aus folgenden Proteasen:
a) Protease, die eine Aminosäuresequenz umfasst, die mindestens 70% Sequenzidentität mit der in SEQ ID NO:1 (B.gibsonii wt) angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt eine der folgenden Aminosäuresubstitutionsvarianten, jeweils bezogen auf die Nummerierung gemäß SEQ ID NO:1, aufweist:
   (i) I43V;
   (ii) M122L, N154S und T156A;
   (iii) M211N und P212D;
   (iv) M211L und P212D;
   (v) G160S;
   (vi) D127P, M211L und P212D;
   (vii) P212H; oder
   (viii) Q12L, M122L und A222S;
b) Protease, die eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80%, bevorzugt zu mindestens 90 %, insbesondere zu 100 % identisch ist und mindestens eine Aminosäuresubstitution an einer der Positionen 9, 15, 66, 212 und 239 in der Zählung gemäß SEQ ID NO:2 aufweist, vorzugsweise ausgewählt aus der Gruppe bestehend aus S9R, A15T, V66A, N212D und Q239R;
c) Protease, die eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80% bevorzugt zu mindestens 90 %, insbesondere zu 100 % identisch ist und eine Aminosäuresubstitution an der Position 97 und eine Insertion von einer Aminosäure zwischen den Aminosäuren an Positionen 97 und 98 in der Zählung gemäß SEQ ID NO:2 aufweist, vorzugsweise ausgewählt aus S97A und/oder S97AD;
d) Protease, die eine Aminosäuresequenz umfasst, die eine zu der in SEQ ID NO:3 (Bacillus alkalophilus PB92) angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer der folgenden Positionen 32, 33, 48-54, 58-62, 94-107, 116, 123-133, 150, 152-156, 158-161, 164, 169, 175-186, 197, 198, 203-216 in der Zählung gemäß SEQ ID NO:3 aufweist, insbesondere mindestens eine Aminosäuresubstitution an einer, zwei, drei oder vier der folgenden Positionen 116, 126, 127, 128 und 160 in der Zählung gemäß SEQ ID NO:3, vorzugsweise mindestens eine, noch bevorzugter mehrere, am bevorzugtesten jede der Aminosäuresubstitution G116V, S126L, P127Q und/oder S128A und/oder
e) Protease, die eine Aminosäuresequenz umfasst, die eine alkalische Protease aus Bacillus lentus DSM 5483 umfasst, insbesondere eine, die eine Aminosäuresequenz aufweist, die zu der in SEQ ID NO:4 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer, zwei, drei oder vier der folgenden Positionen 3, 4, 99 und 199 in der Zählung gemäß SEQ ID NO:4 aufweist, insbesondere die Aminosäuresubstitution R99E oder R99D, sowie optional zusätzlich mindestens eine oder zwei, vorzugsweise alle drei der Aminosäuresubstitutionen S3T, V4I und V199I.

Im Zusammenhang mit der vorliegenden Erfindung bedeutet das Merkmal, dass ein Enzym, insbesondere eine Protease die angegebenen Substitutionen aufweist, dass sie mindestens eine der entsprechenden Aminosäuren an den entsprechenden Positionen enthält, d.h. nicht alle der 10 Positionen anderweitig mutiert oder, beispielsweise durch Fragmentierung des Enzyms, insbesondere der Protease, deletiert sind.

"Variante", wie hierin verwendet, bezieht sich auf natürliche oder artifiziell erzeugte Variationen eines nativen Enzyms, die eine gegenüber der Referenzform abgewandelte Aminosäuresequenz aufweist. Zusätzlich zu den vorstehend erläuterten Aminosäureveränderungen können erfindungsgemäße Enzyme weitere Aminosäureveränderungen, insbesondere Aminosäuresubstitutionen, -insertionen oder -deletionen, aufweisen. Solche Enyzme sind z.B. durch gezielte genetische Veränderung, d.h. durch Mutageneseverfahren, weiterentwickelt und für bestimmte Einsatzzwecke oder hinsichtlich spezieller Eigenschaften (z.B. hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Enzyme oder anderer Polypeptide genutzt werden. Das Ziel ist es, in die bekannten Moleküle gezielte Mutationen wie Substitutionen, Insertionen oder Deletionen einzuführen, um z.B. die Reinigungsleistung von Enzymen zu verbessern. Hierzu können insbesondere die Oberflächenladungen und/oder der isoelektrische Punkt der Moleküle und dadurch ihre Wechselwirkungen mit dem Substrat verändert werden. So kann z.B. die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität des Enzyms erhöht und dadurch seine Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease kann daher im Rahmen der Erfindung zusätzlich weiterentwickelt sein, z.B. hinsichtlich ihrer Stabilität gegenüber Tensiden und/oder Bleichmitteln und/oder anderen Komponenten.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, z.B. einen Stern oder einen Strich, ersetzt oder vor der entsprechenden Position ein Δ angegeben. Beispielsweise beschreibt P14H die Substitution von Prolin an Position 14 durch Histidin, P14HT die Insertion von Threonin nach der Aminosäure Histidin an Position 14 und P14* oder ΔP14 die Deletion von Prolin an Position 14. Diese Nomenklatur ist dem Fachmann auf dem Gebiet der Enzymtechnologie bekannt.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) "Basic local alignment search tool", J. Mol. Biol. 215:403-410 und Altschul et al. (1997) "Gapped BLAST and PSI-BLAST: a new generation of protein database search programs", Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. z.B. Chenna et al. (2003) "Multiple sequence alignment with the Clustal series of programs", Nucleic Acid Res. 31:3497-3500), T-Coffee (vgl. z.B. Notredame et al. (2000) "T-Coffee: A novel method for multiple sequence alignments", J. Mol. Biol. 302:205-217) oder Programme, die auf diesen Programmen beziehungsweise Algorithmen basieren. Ferner möglich sind Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

In einer weiteren Ausführungsform der Erfindung umfasst die erfindungsgemäß eingesetzte Protease eine Aminosäuresequenz, die zu der in SEQ ID NO:1 angegebenen Aminosäuresequenz über deren Gesamtlänge zu mindestens 70% und zunehmend bevorzugt mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die bevorzugt an mindestens einer der Positionen, die den Positionen 12, 43, 122, 127, 154, 156, 160, 211, 212 oder 222 in der Zählung gemäß SEQ ID NO:1 entsprechen, eine oder mehrere der Aminosäuresubstitutionen 12L, 43V, 122L, 127P, 154S, 156A, 160S, 211N, 211L, 212D, 212H oder 222S aufweist.

In einer weiteren Ausführungsform der Erfindung umfasst die erfindungsgemäß eingesetzte Protease eine Aminosäuresequenz, die zu der in SEQ ID NO:2 angegebenen Aminosäuresequenz über deren Gesamtlänge zu mindestens 81% und zunehmend bevorzugt mindestens 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die bevorzugt mindestens eine Aminosäuresubstitution an einer der Positionen 9, 15, 66, 212 und 239 in der Zählung gemäß SEQ ID NO:2 entsprechen, eine oder mehrere Aminosäuresubstitionen aufweisen, vorzugsweise ausgewählt aus der Gruppe bestehend aus 9R, 15T, 66A, 212D oder 239R.

In einer weiteren Ausführungsform der Erfindung umfasst die erfindungsgemäß eingesetzte Protease eine Aminosäuresequenz, die zu der in SEQ ID NO:2 angegebenen Aminosäuresequenz über deren Gesamtlänge zu mindestens 81% und zunehmend bevorzugt mindestens 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die eine Aminosäuresubstitution an der Position 97 und eine Insertion von einer Aminosäure zwischen den Aminosäuren an Positionen 97 und 98 in der Zählung gemäß SEQ ID NO:2 aufweist, vorzugsweise ausgewählt aus 97A und/oder 97AD.

In einer weiteren Ausführungsform der Erfindung umfasst die erfindungsgemäß eingesetzte Protease eine Aminosäuresequenz, die zu der in SEQ ID NO:3 angegebenen Aminosäuresequenz über deren Gesamtlänge zu mindestens 81% und zunehmend bevorzugt mindestens 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die bevorzugt an mindestens einer der Positionen, die den Positionen 32, 33, 48-54, 58-62, 94-107, 116, 123-133, 150, 152-156, 158-161, 164, 169, 175-186, 197, 198, 203-216 in der Zählung gemäß SEQ ID NO:3 entsprechen, insbesondere mindestens eine Aminosäuresubstitution an einer, zwei, drei oder vier der folgenden Positionen 116, 126, 127, 128 und 160 in der Zählung gemäß SEQ ID NO:3, vorzugsweise mindestens eine, noch bevorzugter mehrere, am bevorzugtesten jede der Aminosäuresubstitution 116V, 126L, 127Q und/oder 128A aufweist.

In einer weiteren Ausführungsform der Erfindung umfasst die erfindungsgemäß eingesetzte Protease eine Aminosäuresequenz, die zu der in SEQ ID NO:4 angegebenen Aminosäuresequenz über deren Gesamtlänge zu mindestens 81% und zunehmend bevorzugt mindestens 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die bevorzugt an mindestens einer der Positionen, die den Positionen 3, 4, 99 und 199 in der Zählung gemäß SEQ ID NO:4 insbesondere die Aminosäuresubstitution 99E oder 99D, sowie optional zusätzlich mindestens eine oder zwei, vorzugsweise alle drei der Aminosäuresubstitutionen 3T, 4I und 199I aufweist.

Gemäß einer weiteren besonders bevorzugten Ausführungsform der vorliegenden Erfindung beträgt die Menge aller Proteasen, bezogen auf die Menge an aktivem Enzymprotein, welche pro Spülgang hinzudosiert wird, bevorzugt 0,001 bis 1000 mg/job, weiter bevorzugt 0,1 bis 600 mg/job und noch weiter bevorzugt 1,0 bis 400 mg/job.

Besonders bevorzugt umfasst die wenigstens eine Gelphase als ein Enzym (ggf. neben weiteren Enzymen) eine Protease, die eine Aminosäuresequenz umfasst, die mindestens 70% Sequenzidentität mit der in SEQ ID NO:1 (B.gibsonii wt) angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt eine der folgenden Aminosäuresubstitutionsvarianten, jeweils bezogen auf die Nummerierung gemäß SEQ ID NO:1, aufweist:
(i) I43V;
(ii) M122L, N154S und T156A;
(iii) M211N und P212D;
(iv) M211L und P212D;
(v) G160S;
(vi) D127P, M211L und P212D;
(vii) P212H; oder
(viii) Q12L, M122L und A222S.

Ganz besonders bevorzugt enthält das erfindungsgemäße Reinigungsmittel 0,001 bis 1000 mg weiter bevorzugt 0,1 bis 600 mg und noch weiter bevorzugt 1,0 bis 400 mg pro Reinigungsmittelportion, bevorzugt in der Gelphase des Reinigungsmittels, einer Protease, die eine Aminosäuresequenz umfasst, die mindestens 70% Sequenzidentität mit der in SEQ ID NO:1 (B.gibsonii wt) angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt eine der folgenden Aminosäuresubstitutionsvarianten, jeweils bezogen auf die Nummerierung gemäß SEQ ID NO:1, aufweist:
(i) I43V;
(ii) M122L, N154S und T156A;
(iii) M211N und P212D;
(iv) M211L und P212D;
(v) G160S;
(vi) D127P, M211L und P212D;
(vii) P212H; oder
(viii) Q12L, M122L und A222S.

Gemäß einem anderen Gegenstand der Erfindung ist es ebenfalls möglich, dass in einem Reinigungsmittel, insbesondere einer Reinigungsmitteleinmalportion, welche mindestens eine Gelphase und mindestens eine weitere, bevorzugt eine feste Phase, umfassend körnige Gemenge, insbesondere Pulver, umfasst, eine Protease aus der Gruppe der im direkt vorstehenden Abschnitt genannten Proteasen, nicht in die Gelphase eingearbeitet werden, sondern anteilsweise oder vollständig in die mindestens eine weitere, bevorzugt mindestens eine feste Phase, insbesondere bevorzugt mindestens eine weitere feste Phase, umfassend ein körniges Gemenge eingearbeitet werden kann. Die einzusetzenden Mengen bezogen auf die einzusetzende Reinigungsmittelportion bleiben dabei unverändert. Die vorstehend bzw. im weiteren beschriebenen Ausführungsformen für die Gelphase sowie die weitere, bevorzugt feste Phase, gelten analog auch für diese Ausführungsform.

Gemäß einer weiteren bevorzugten Ausführungsform der vorliegenden Erfindung beträgt der Gewichtsanteil aller Protease, bezogen auf die Menge an aktivem Enzymprotein aller Proteasen, am Gesamtgewicht des gesamten Reinigungsmittels 0,005 bis 5 Gew.-%, bevorzugt 0,01 bis 2,0 Gew.-%, weiter bevorzugt 0,05 bis 1,5 Gew.-% und noch weiter bevorzugt 0,01 bis 1,0 Gew.-%.

Gemäß einer weiteren bevorzugten Ausführungsform der vorliegenden Erfindung beträgt der Gewichtsanteil aller Proteasen, bezogen auf die Menge an aktivem Enzymprotein, in der mindestens einen gelförmigen Phase, bezogen auf das Gesamtgewicht der gelförmigen Phase(n), 0,0005 bis 20,0 Gew.-%, bevorzugt 0,001 bis 15 Gew.-%, weiter bevorzugt 0,01 bis 10,0 Gew.-% und noch weiter bevorzugt 0,1 bis 7,5 Gew.-%.

Gemäß einer weiteren bevorzugten Ausführungsform der vorliegenden Erfindung beträgt die Menge aller Proteasen, bezogen auf die Menge an aktivem Enzymprotein, welche pro Spülgang hinzudosiert wird, bevorzugt 0,001 bis 1000 mg/job, weiter bevorzugt 0,1 bis 600 mg/job und noch weiter bevorzugt 1,0 bis 400 mg/job.

Gemäß einer weiteren bevorzugten Ausführungsform umfasst die wenigstens eine Gelphase mindestens eine Amylase, ggf. zusätzlich zu anderen Enzymen, insbesondere Protease(n). Erfindungsgemäß bevorzugte Amylasen sind α-Amylasen. Entscheidend dafür, ob ein Enzym eine α-Amylase im Sinne der Erfindung ist, ist deren Fähigkeit zur Hydrolyse von α-(1-4)-Glykosidbindungen in Poylsacchariden, insbesondere Amylose und Stärke. Dadurch bewirken sie den Abbau stärkehaltiger Anschmutzungen auf dem Reinigungsgut. Als Spaltprodukte entstehen dabei Dextrine und daraus Maltose, Glucose und verzweigte Oligosaccharide.

Beispiele für Amylasen sind die α-Amylasen aus *Bacillus licheniformis, Bacillus amyloliquefaciens* oder *Bacillus stearothermophilus* sowie insbesondere auch deren für den Einsatz in Wasch- oder Reinigungsmitteln verbesserte Weiterentwicklungen. Das Enzym aus *Bacillus licheniformis* ist von dem Unternehmen Novozymes unter dem Namen Termamyl® und von dem Unternehmen Danisco/Genencor unter dem Namen Purastar® ST erhältlich. Weiterentwicklungsprodukte dieser α-Amylase sind von dem Unternehmen Novozymes unter den Handelsnamen Duramyl® und Termamyl® ultra, von dem Unternehmen Danisco/Genencor unter dem Namen Purastar® OxAm und von dem Unternehmen Daiwa Seiko Inc. als Keistase® erhältlich. Die α-Amylase von *Bacillus amyloliquefaciens* wird von dem Unternehmen Novozymes unter dem Namen BAN® vertrieben, und abgeleitete Varianten von der α-Amylase aus *Bacillus stearothermophilus* unter den Namen BSG® und Novamyl®, ebenfalls von dem Unternehmen Novozymes. Des Weiteren sind für diesen Zweck die α-Amylase aus *Bacillus sp.* A 7- 7 (DSM 12368) und die Cyclodextrin-Glucanotransferase (CGTase) aus *Bacillus agaradherens* (DSM 9948) hervorzuheben. Ferner sind die amylolytischen Enzyme einsetzbar, die in den internationalen Patentanmeldungen WO2003002711, WO2003054177 und WO2007079938 offenbart sind, auf deren Offenbarung daher ausdrücklich verwiesen wird bzw. deren diesbezüglicher Offenbarungsgehalt daher ausdrücklich in die vorliegende Patentanmeldung mit einbezogen wird. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus *Aspergillus niger* und *A. oryzae* geeignet. Weitere vorteilhaft einsetzbare Handelsprodukte sind z.B. die Amylase-LT® sowie Stainzyme® oder Stainzyme ultra® bzw. Stainzyme plus® ebenfalls von dem Unternehmen Novozymes. Auch durch Punktmutationen erhältliche Varianten dieser Enzyme können erfindungsgemäß eingesetzt werden.

Die erfindungsgemäß in den Wasch- oder Reinigungsmitteln eingesetzte α-Amylase ist vorzugsweise ausgewählt aus:
i. einer α-Amylase, die eine Aminosäuresequenz umfasst, die zu der in SEQ ID NO:5 angegebenen Aminosäuresequenz über deren Gesamtlänge zu mindestens 80%, bevorzugt mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Aminosäuresubstitution an einer der Positionen 172, 202, 208, 255 und 261 in der Zählung gemäß SEQ ID NO:5 aufweist, vorzugsweise ausgewählt aus der aus M202L, M202V, M202S, M202T, M202I, M202Q, M202W, S255N, R172Q und Kombinationen davon bestehenden Gruppe; und/oder
ii. einer α-Amylase, die eine Aminosäuresequenz umfasst, die zu der in SEQ ID NO:6 angegebenen Aminosäuresequenz über deren Gesamtlänge zu mindestens 60%, bevorzugt mindestens 61%, 62%, 63%, 64%, 65%, 66%, 67%, 68%, 69%, 70% 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine Deletion an einer der Positionen 183 und 184 in der Zählung gemäß SEQ ID NO:6 aufweist, vorzugsweise mindestens eine Aminosäuresubstitution an einer der Positionen 9, 26, 149, 182, 186, 202, 257, 295, 299, 323, 339 and 345, besonders bevorzugt aus der aus R118K, D183*, G184*, N195F, R320K, R458K und Kombinationen davon bestehenden Gruppe ausgewählt; und/oder
iii. einer α-Amylase, die eine Aminosäuresequenz umfasst, die zu der in SEQ ID NO:7 angegebenen Aminosäuresequenz über deren Gesamtlänge zu mindestens 90% identisch ist und optional mindestens eine Substitution und/oder Deletion an einer der Positionen 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 in der Zählung gemäß SEQ ID NO:7, aufweist.

In verschiedenen Ausführungsformen der Erfindung umfasst die Amylase eine Aminosäuresequenz umfasst, die zu der in SEQ ID NO:5 angegebenen Aminosäuresequenz über deren Gesamtlänge zu mindestens 80%, bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Aminosäuresubstitution an einer der Positionen 172, 202, 208, 255 und 261 in der Zählung gemäß SEQ ID NO:5 aufweist, vorzugsweise ausgewählt aus der aus M202L, M202V, M202S, M202T, M202I, M202Q, M202W, S255N, R172Q und Kombinationen davon bestehenden Gruppe. Bevorzugt werden Amylasen eingesetzt, die an zwei, bevorzugt drei, der vorstehend genannten Positionen eine Aminosäuresubstitution aufweisen, insbesondere eine Substitution an Position 202 ausgewählt aus M202L, M202V, M202S, M202T, M202I, M202Q, M202W, eine Substitution an Position 255, insbesondere S255N, und eine Substitution an Position 172, insbesondere R172Q. Ganz besonders bevorzugt sind die M202L und M202T Mutanten.

In verschiedenen Ausführungsformen der Erfindung umfasst die Amylase eine Aminosäuresequenz, die zu der in SEQ ID NO:6 angegebenen Aminosäuresequenz über deren Gesamtlänge zu mindestens 60%, bevorzugt mindestens 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine Deletion an einer der Positionen 183 und 184 in der Zählung gemäß SEQ ID NO:6 aufweist, vorzugsweise mindestens eine Aminosäuresubstitution an einer der Positionen 9, 26, 149, 182, 186, 202, 257, 295, 299, 323, 339 and 345, besonders bevorzugt aus der aus R118K, D183*, G184*, N195F, R320K, R458K und Kombinationen davon bestehenden Gruppe ausgewählt. In verschiedenen bevorzugten Ausführungsformen weist die Amylase in der Zählung gemäß SEQ ID NO:6 Aminosäuresubstitutionen an drei oder mehr der Positionen 9, 26, 149, 182, 186, 202, 257, 295, 299, 323, 339 and 345 und optional eine oder mehrere, vorzugsweise alle, der Substitutionen und/oder Deletionen an den Positionen: 118, 183, 184, 195, 320 und 458, besonders bevorzugt R118K, D183*, G184*, N195F, R320K und/oder R458K, auf. In besonders bevorzugten Ausführungsformen weist die Amylase in der Zählung gemäß SEQ ID NO:6 folgende Aminosäuresubstitutionen und/oder Deletionen auf: M9L + M323T; M9L + M202L/T/V/I + M323T; M9L + N195F + M202L/T/V/I + M323T; M9L + R118K + D183* + G184* + R320K + M323T + R458K; M9L + R118K + D183* + G184* + M202L/T/V/I + R320K + M323T + R458K; M9L + G149A + G182T + G186A + M202L + T257I + Y295F + N299Y + M323T + A339S + E345R; M9L + G149A + G182T + G186A + M202I + T257I + Y295F + N299Y + M323T + A339S + E345R; M9L + R118K + G149A + G182T + D183* + G184* + G186A + M202L + T257I + Y295F + N299Y + R320K + M323T + A339S + E345R + R458K; M9L + R118K + G149A + G182T + D183* + G184* + G186A + N195F + M202L + T257I + Y295F + N299Y + R320K + M323T + A339S + E345R + R458K; M9L + R118K + G149A + G182T + D183* + G184* + G186A + M202I + T257I + Y295F + N299Y + R320K + M323T + A339S + E345R + R458K; M9L + R118K + D183* + D184* + N195F + M202L + R320K + M323T + R458K; M9L + R118K + D183* + D184* + N195F + M202T + R320K + M323T + R458K; M9L + R118K + D183* + D184* + N195F + M202I + R320K + M323T + R458K; M9L + R118K + D183* + D184* + N195F + M202V + R320K + M323T + R458K; M9L + R118K + N150H + D183* + D184* + N195F + M202L + V214T + R320K + M323T + R458K; oder M9L + R118K + D183* + D184* + N195F + M202L + V214T + R320K + M323T + E345N + R458K.

Eine besonders bevorzugte Amylase ist die Variante, die unter dem Handelnamen Stainzyme Plus™ kommerziell erhältlich ist (Novozymes A/S, Bagsvaerd, Dänemark).

In verschiedenen Ausführungsformen der Erfindung umfasst die Amylase eine Aminosäuresequenz umfasst, die zu der in SEQ ID NO:7 angegebenen Aminosäuresequenz über deren Gesamtlänge zu mindestens 90%, bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Substitution und/oder Deletion an einer der Positionen 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 in der Zählung gemäß SEQ ID NO:7, aufweist. Diesbezüglich bevorzugte Aminosäuresubstitutionen umfassen: E260A/D/C/Q/L/M/F/P/S/W/V/G/H/I/K/N/R/T/Y, G304R/K/E/Q, W140Y/F, W189E/G/T, D134E, F262G/P, W284D/H/F/Y/R, W347H/F/Y, W439R/G, G476E/Q/R/K, G477E/Q/K/M/R, N195F/Y, N197F/L, Y198N, Y200F, Y203F, I206H/L/N/F/Y, H210Y, E212V/G, V213A, M116T, Q129L, G133E, E134Y, K142R, P146S, G147E, G149R, N151R, Y152H, Q169E, N174R, A186R, Y243F, S244Q, G303V, R320N, R359I, N418D und A447V.

In verschiedenen bevorzugten Ausführungsformen können erfindungsgemäße Reinigungsmittel mindestens eine zweite Amylase und/oder mindestens eine zweite Protease enthalten, wobei die zweite Amylase unterschiedlich zur ersten Amylase ist und aus der oben genannten Gruppe ausgewählt ist und wobei die zweite Protease unterschiedlich zu ersten Protease ist.

Wenn zwei Amylasen enthalten sind, können diese vorzugsweise im Massenverhältnis von 50:1 bis 1:50, vorzugsweise 30:1 bis 1:10 (jeweils bezogen auf die Menge Aktivprotein Amylase 1 zu Amylase 2) eingesetzt werden. Es ist insbesondere bevorzugt, eine erste Amylase im Verhältnis zu einer zweiten Amylase von 20:1 bis 2:1, bevorzugt 15:1 bis 3:1, besonders bevorzugt 12:1 bis 5:1, beispielsweise 10:1 einzusetzen.

Gemäß einer bevorzugten Ausführungsform der vorliegenden Erfindung beträgt der Gewichtsanteil der mindestens einen Amylase, bezogen auf die Menge an aktivem Enzymprotein aller Amylasen, am Gesamtgewicht des Reinigungsmittels 0,0005 bis 2 Gew.-%, bevorzugt 0,001 bis 1,5 Gew.-%, weiter bevorzugt 0,005 bis 1,0 Gew.-% und noch weiter bevorzugt 0,001 bis 0,8 Gew.-%.

Erfindungsgemäß einsetzbar sind weiterhin Lipasen oder Cutinasen, insbesondere wegen ihrer Triglycerid-spaltenden Aktivitäten, aber auch, um aus geeigneten Vorstufen in situ Persäuren zu erzeugen. Hierzu gehören beispielsweise die ursprünglich aus Humicola lanuginosa (Thermomyces lanuginosus) erhältlichen, beziehungsweise weiterentwickelten Lipasen, insbesondere solche mit dem Aminosäureaustausch in den Positionen D96LT213R und/oder N233R, besonders bevorzugt alle der Austausche D96L, T213R und N233R.

Weiterhin können Enzyme eingesetzt werden, die unter dem Begriff Hemicellulasen zusammengefasst werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen.

Zur Erhöhung der bleichenden Wirkung können erfindungsgemäß Oxidoreduktasen, beispielsweise Oxidasen, Oxygenasen, Katalasen, Peroxidasen, wie Halo-, Chloro-, Bromo-, Lignin-, Glucose- oder Mangan-Peroxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren). Ein Protein und/oder Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Reinigungsmittel können zu diesem Zweck Stabilisatoren enthalten; die Bereitstellung derartiger Mittel stellt eine bevorzugte Ausführungsform der vorliegenden Erfindung dar.

Reinigungsaktive Proteasen und Amylasen werden in der Regel nicht in Form des reinen Proteins sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, vorteilhafterweise möglichst konzentriert, wasserarm und/oder mit Stabilisatoren oder weiteren Hilfsmitteln versetzt.

Alternativ können die Enzyme für die wenigstens eine Gelphase, bevorzugt feste Gelphase, verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Wie aus den vorherigen Ausführungen ersichtlich, bildet das Enzym-Protein nur einen Bruchteil des Gesamtgewichts üblicher Enzym-Zubereitungen. Erfindungsgemäß eingesetzte Protease- und Amylase-Zubereitungen enthalten zwischen 1 und 40 Gew.-%, bevorzugt zwischen 2 und 30 Gew.-%, besonders bevorzugt zwischen 3 und 25 Gew.-% des Enzymproteins. Bevorzugt werden insbesondere solche Reinigungsmittel, die, jeweils bezogen auf ihr Gesamtgewicht, 0,1 bis 12 Gew.-%, vorzugsweise 0,2 bis 10 Gew.-% und insbesondere 0,5 bis 8 Gew.-% der jeweiligen Enzym-Zubereitungen enthalten.

Unter einer gelförmigen Phase, im weiteren auch Gelphase genannt, ist erfindungsgemäß eine Zusammensetzung/Phase zu verstehen, welche ein intern strukturierendes Netzwerk aufweist. Dieses intern strukturierende (räumliche) Netzwerk wird durch die Dispersion eines festen, aber verteilten Stoff mit langen oder stark verzweigten Teilchen und/oder Gelbildner, in mindestens einer Flüssigkeit (die mindestens eine Flüssigkeit ist flüssig bei 20 °C) gebildet. Solche Gelphasen verhalten sich thermoreversibel.

Diese Gelphase kann beispielsweise fließfähig sein oder formstabil. Erfindungsgemäß bevorzugt ist die gelförmige Phase allerdings bei Raumtemperatur formstabil. Bei der Herstellung wird der Gelbildner, bevorzugt Xanthan, Gelatine oder Polyvinylalkohol und/oder dessen Derivate mit einem Lösungsmittel, vorzugsweise organischem Lösungsmittel, vorzugsweise einem oder mehreren mehrwertigen Alkohol(en) in Kontakt gebracht. Hierdurch erhält man eine fließfähige Mischung, welche in eine gewünschte Form gebracht werden kann. Nach einem gewissen Zeitraum erhält man eine Gelphase, die in der vorgegebenen Form bleibt, also formstabil ist. Dieser Zeitraum, die Erstarrungszeit, beträgt vorzugsweise 15 Minuten oder weniger, vorzugsweise 10 Minuten oder weniger, besonders bevorzugt 5 Minuten oder weniger. Dabei gibt die wenigstens eine Gelphase auf Druck nach, verformt sich hierdurch jedoch nicht, sondern kehrt nach Wegfall des Drucks in den Ausgangszustand zurück. Die wenigstens eine Gelphase ist vorzugsweise elastisch, insbesondere linear-elastisch.

Die wenigstens eine Gelphase ist bevorzugt ein Formkörper. Ein Formkörper ist ein einzelner Körper, der sich in seiner aufgeprägten Form selbst stabilisiert. Dieser formstabile Körper wird aus einer Formmasse (z.B. eine Zusammensetzung) dadurch gebildet, dass diese Formmasse gezielt in eine vorgegebene Form gebracht wird, z.B. durch Gießen einer flüssigen Zusammensetzung in eine Gussform und anschließendem Aushärten der flüssigen Zusammensetzung, z.B. im Rahmen eines Sol-Gel-Prozesses.

An Formulierungen der wenigstens einen Gelphase werden bestimmte Mindestanforderungen gestellt. So muss, wie bereits ausgeführt, die Gelphase innerhalb einer möglichst kurzen Zeit erstarren. Lange Erstarrungszeiten würden zu einer langen Produktionsdauer und damit zu hohen Kosten führen. Erfindungsgemäß bedeutet Erstarrungszeit der Zeitraum, innerhalb dessen bei der Herstellung die wenigstens eine Gelphase von einem fließfähigen in einen bei Raumtemperatur nicht-fließfähigen, formstabilen Zustand übergeht. Unter Raumtemperatur ist dabei eine Temperatur von 20 °C zu verstehen.

Die wenigstens eine Gelphase ist bevorzugt eine feste Gelphase. Sie ist dabei schnittfest. Sie kann beispielsweise mit einem Messer nach der Erstarrung geschnitten werden, ohne dass sie, außer dem durchgeführten Schnitt, weiter zerstört wird.

Die wenigstens eine Gelphase ist zudem vorzugsweise transluzent (durchscheinend) oder transparent, wodurch sich ein guter optischer Eindruck ergibt. Bevorzugt liegt die Transmission der Gelphase (ohne Farbstoff) in einem Bereich zwischen 100 % und 20 %, zwischen 100 % und 30 %, insbesondere zwischen 100 % und 40 %. Zur Messung der Lichtdurchlässigkeit (Transmission) wurde die Durchgängigkeit in % bei 600 nm gegen Wasser als Referenz bei 20 °C ermittelt. Die Masse wurde dafür in die vorgesehenen 11 mm Rundküvetten gegossen und nach 12 h Lagerzeit bei Raumtemperatur in einem LICO 300 Farbmesssystem nach Lange vermessen.

Die wenigstens eine Gelphase ist wasserarm. Wasserarm im Sinne der vorliegenden Erfindung bedeutet das geringe Mengen an Wasser zur Herstellung der wenigstens einen Gelphase eingesetzt werden können. Der Anteil an Wasser in der Gelphase beträgt insbesondere 20 Gew.-% oder weniger, bevorzugt 15 Gew.-% oder weniger, besonders 12 Gew.-%, oder weniger, insbesondere zwischen 10 und 5 Gew.-%. Die Angaben in Gew.-% beziehen sich auf das Gesamtgewicht der Gelphase. Das hat den Vorteil, dass die geringen Mengen Wasser in Kombination PVOH struktur- bzw. gelbildend wirken können.

Gemäß einer bevorzugten Ausführungsform ist die wenigstens eine Gelphase im Wesentlichen wasserfrei. Dies bedeutet, dass die Gelphase bevorzugt im Wesentlichen frei von Wasser ist. "Im Wesentlichen frei" bedeutet hier, dass in der Gelphase geringe Mengen an Wasser enthalten sein können. Dieses Wasser kann beispielsweise durch ein Lösungsmittel oder als Kristallwasser oder auf Grund von Reaktionen von Bestandteilen der Phase miteinander in die Phase eingebracht werden. Es wird jedoch bevorzugt nur geringe Mengen, insbesondere kein Wasser als Lösungsmittel zur Herstellung der Gelphase eingesetzt. Der Anteil an Wasser in der Gelphase beträgt in dieser Ausführungsform 4,9 Gew.-% oder weniger, 4 Gew.-% oder weniger, bevorzugt 2 Gew.-% oder weniger, insbesondere 1 Gew.-% oder weniger, besonders 0,5 Gew.-% oder weniger, insbesondere 0,1 Gew.-% oder 0,05 Gew.-% oder weniger. Die Angaben in Gew.-% beziehen sich dabei auf das Gesamtgewicht der Gelphase.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist. Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden. "Mindestens ein", wie hierin verwendet, bedeutet 1 oder mehr, d.h. 1, 2, 3, 4, 5, 6, 7, 8, 9 oder mehr. Bezogen auf einen Inhaltsstoff bezieht sich die Angabe auf die Art des Inhaltsstoffs und nicht auf die absolute Zahl der Moleküle. "Mindestens ein Bleichkatalysator" bedeutet somit beispielsweise mindestens eine Art von Bleichkatalysator, d.h. dass eine Art von Bleichkatalysator oder eine Mischung mehrerer verschiedener Bleichkatalysatoren gemeint sein kann. Zusammen mit Gewichtsangaben bezieht sich die Angabe auf alle Verbindungen der angegebenen Art, die in der Zusammensetzung/Mischung enthalten sind, d.h. dass die Zusammensetzung über die angegebene Menge der entsprechenden Verbindungen hinaus keine weiteren Verbindungen dieser Art enthält.

Wenn hierin auf Molmassen Bezug genommen wird, beziehen sich diese Angaben immer auf die zahlenmittlere Molmasse Mₙ, sofern nicht explizit anders angeben. Das Zahlenmittel der Molmasse kann beispielsweise mittels Gel-Permeations-Chromatographie (GPC) gemäß DIN 55672-1:2007-08 mit THF als Eluent bestimmt werden. Die massenmittlere Molmasse M_{w} kann ebenfalls mittels GPC bestimmt werden, wie für Mₙ beschrieben.

Alle Prozentangaben, die im Zusammenhang mit den hierin beschriebenen Zusammensetzungen gemacht werden, beziehen sich, sofern nicht explizit anders angegeben auf Gew.-%, jeweils bezogen auf die betreffende Mischung bzw. Phase.

Weiterhin muss die Gelphase lagerstabil sein, und zwar bei üblichen Lagerbedingungen. Die erfindungsgemäße Gelphase ist Bestandteil eines Reinigungsmittels. Reinigungsmittel werden in einem Haushalt üblicherweise über einen gewissen Zeitraum gelagert. Die Lagerung erfolgt üblicherweise in der Nähe der Wasch- bzw. Spülmaschine. Für eine solche Lagerung sollte die Gelphase stabil sein. Somit sollte die Gelphase insbesondere auch nach einer Lagerzeit von beispielsweise 4 bis 12 Wochen, insbesondere 10 bis 12 Wochen oder länger bei einer Temperatur von bis zu 40°C, besonders bei 30 °C, insbesondere bei 25 °C oder bei 20 °C stabil sein und sich in dieser Zeit nicht verformen oder sonst wie in der Konsistenz ändern.

Liegen die Gelphase und eine feste, insbesondere eine Pulverphase in direktem Kontakt miteinander vor, dringt die Gelphase in der Lagerzeit von 4 Wochen bei 25 °C bevorzugt maximal 1 mm in die Zwischenräume der unmittelbar unterliegenden Pulverphase ein.

Nachteilig wäre eine Volumenänderung oder Schrumpfung während der Lagerung, da hierdurch die Akzeptanz des Produktes beim Verbraucher gering wäre. Auch ein Austritt von Flüssigkeit oder das Ausschwitzen von Bestandteilen aus der Gelphase ist unerwünscht. Auch hier ist zum einen der optische Eindruck von Relevanz. Durch den Austritt von Flüssigkeit, wie beispielsweise Lösungsmittel, kann die Stabilität der Gelphase beeinflusst werden, so dass die Bestandteile nicht mehr stabil enthalten sind und dadurch auch die Wasch- bzw. Reinigungswirkung beeinflusst werden kann.

Gemäß einer bevorzugten Ausführungsform enthalten die erfindungsgemäßen Reinigungsmittel, bevorzugt Geschirrspülmittel, insbesondere maschinellen Geschirrspülmittel in der gelförmigen Phase (Gelphase) einen Gelbildner, bevorzugt ausgewählt aus Gelatine, Xanthan und/oder Polyvinylalkohol, insbesondere Gelatine oder Polyvinylalkohol, besonders bevorzugt Polyvinylalkohol, in einer Menge von 4 bis 40, insbesondere von 6 bis 30 Gew.-%, besonders bevorzugt in einer Menge von 7 bis 24 Gew.-%, ganz besonders bevorzugt 8 bis 22 Gew.-%, insbesondere beispielsweise 14 bis 20 Gew.-%, jeweils bezogen auf das Gesamtgewicht der gelförmigen Phase.

Erfindungsgemäß besonders bevorzugt umfasst die wenigstens eine Gelphase PVOH (Polyvinylalkohol) und/oder deren Derivate. Polyvinylalkohole sind thermoplastische Kunststoffe, die als weißes bis gelbliches Pulver meistens durch Hydrolyse von Polyvinylacetat hergestellt werden. Polyvinylalkohol (PVOH) ist beständig gegen fast alle wasserfreien organischen Lösemittel. Bevorzugt sind Polyvinylalkohole mit einer Molmasse von 30.000 bis 60.000 g/mol. Als Derivate des PVOH sind im Sinne der Erfindung bevorzugt Copolymere von Polyvinylalkohol mit anderen Monomeren, insbesondere Copolymer mit anionischen Monomeren. Als anionische Monomere sind bevorzugt geeignet Vinylessigsäure, Alkylacrylate, Maleinsäure und deren Derivate, insbesondere Monoalkylmaleate (insbesondere Monomethylmaleat), Dialkylmaleate (insbesondere Dimethylmaleat), Maleinsäureanhydrid, Fumarsäure und deren Derivate, insbesondere Monoalkylfumarat (insbesondere Monomethylfumarat), Dialkylfumarat (insbesondere Dimethylfumarat), Fumarsäureanhydrid, Itaconsäure und deren Derivate, insbesondere Monomethylitaconat, Dialkylitaconat, Dimethylitaconat, Itaconsäureanhydrid, Citraconsäure (Methylmaleinsäure) und deren Derivate, Monoalkylcitraconsäure (insbesondere Methylcitraconat), Dialkylcitraconsäure (Dimethylcitraconat), Citraconsäureanhydrid, Mesaconsäure (Methylfumarsäure) und deren Derivate, Monoalkylmesaconat, Dialkylmesaconat, Mesaconsäureanhydrid, Glutaconsäure und deren Derivate, Monoalkylglutaconat, Dialkylglutaconat, Glutaconsäureanhydrid, Vinylsulfonsäure, Alkylsulfonsäure, Ethylensulfonsäure, 2-Acrylamido-1-methylpropansulfonsäure, 2-acrylamido-2-methylpropansulfonsäure, 2-Methylacrylamido-2-methylpropansulfonsäure, 2-Sulfoethylacrylat sowie deren Kombinationen sowie die Alkalimetallsalze oder Ester der vorstehend genannten Monomere.

Besonders bevorzugt sind als Derivate von PVOH solche, die ausgewählt sind aus Copolymeren von Polyvinylalkohol mit einem Monomer insbesondere ausgewählt aus der Gruppe der Monoalkylmaleate (insbesondere Monomethylmaleat), Dialkylmaleate (insbesondere Dimethylmaleat), Maleinsäureanhydrid, und deren Kombinationen, sowie die Alkalisalze oder Ester der vorstehend genannten Monomere. Für die geeigneten Molmassen gilt das für Polyvinylalkohole selbst angegebenen Werte. Im Rahmen der vorliegenden Erfindung ist es bevorzugt, dass die wenigstens eine Gelphase einen Polyvinylalkohol und/oder dessen Derivate, bevorzugt Polyvinylalkohol, umfasst, dessen Hydrolysegrad vorzugsweise 70 bis 100 Mol-%, insbesondere 80 bis 90 Mol-%, besonders bevorzugt 81 bis 89 Mol-% und vor allem 82 bis 88 Mol-% beträgt.

Besonders bevorzugt sind Polyvinylalkohole, die als weiß-gelbliche Pulver oder Granulate mit Polymerisationsgraden im Bereich von ca. 100 bis 2500 (Molmassen von ca. 4000 bis 100.000 g/mol) und Hydrolysegraden von 80 bis 99 Mol%, bevorzugt von 85 bis 90 Mol%, insbesondere von 87 bis 89 Mol%, beispielsweise 88 Mol% aufweisen, die dementsprechend noch einen Restgehalt an Acetyl-Gruppen enthalten.

PVOH Pulver, mit den vorstehend genannten Eigenschaften, welche zum Einsatz in der wenigstens einen Gelphasen geeignet sind, werden beispielsweise unter der Bezeichnung Mowiol® oder Poval® von Kuraray vertrieben. Geeignet ist z.B. auch Exceval® AQ4104 von Kuraray. Besonders geeignet sind Mowiol C30, die Poval® Qualitäten, insbesondere die Qualitäten 3-83, 3-88, 6-88, 4-85, und besonders bevorzugt 4-88, ganz besonders bevorzugt Poval 4-88 S2 sowie Mowiol® 4-88 von Kuraray.

Die Wasserlöslichkeit von Polyvinylalkohol kann durch Nachbehandlung mit Aldehyden (Acetalisierung) oder Ketonen (Ketalisierung) verändert werden. Als besonders bevorzugt und aufgrund ihrer ausgesprochen guten Kaltwasserlöslichkeit besonders vorteilhaft haben sich hierbei Polyvinylalkohole herausgestellt, die mit den Aldehyd- bzw. Ketogruppen von Sacchariden oder Polysacchariden oder Mischungen hiervon acetalisiert bzw. ketalisiert werden. Als äußerst vorteilhaft einzusetzen sind die Reaktionsprodukte aus Polyvinylalkohol und Stärke. Weiterhin lässt sich die Wasserlöslichkeit durch Komplexierung mit Ni- oder Cu-Salzen oder durch Behandlung mit Dichromaten, Borsäure, Borax verändern und so gezielt auf gewünschte Werte einstellen.

Gemäß einer bevorzugten Ausführungsform wird das mindestens Enzym bzw. werden die Enzyme in die Gelphase, insbesondere die Gelphase mit Gelbildner nicht als flüssige Enzymzubereitung eingebracht. Besonders bevorzugt ist es, dass die Enzyme nicht in solche Gelphasen eingebracht werden, in denen der Gelbildner ausgewählt ist aus Gelatine, Xanthan und/oder Polyvinylalkohol, insbesondere Gelatine oder Polyvinylaklohol, besonders bevorzugt Polyvinylalkohol. Die Einbringung flüssiger Enzymzubereitungen in noch durch die Herstellung erwärmte Gelphasen, insbesondere bei der Herstellung von PVOH-haltigen Gelphasen werden hohe Temperaturen verwendet, die dazu führen, dass die Stabilität der Enzyme in der flüssigen Enzymzubereitung stark vermindert wird. Eine Verminderung der Stabilität bedeutet insbesondere, dass die Aktivität der Enzyme deutlich gegenüber der Ausgangszubereitung herabgesetzt ist. Diese Verminderung der Stabilität kann beispielsweise durch die Bestimmung der Restenzymaktivität oder auch durch den Vergleich der Reinigungsleistung der entsprechenden Enzyme, insbesondere gemäß IKW-Standards gemessen werden.

Überraschenderweise hat sich gezeigt, dass PVOH besonders geeignet sind, Gelphasen herzustellen, die den oben gezeigten Anforderungen genügen. Besonders bevorzugt ist daher wenigstens eine Gelphase, die neben mindestens einem Enzym, insbesondere Enzymgranulat, bevorzugt ausgewählt aus der Gruppe Proteasen und/oder Amylasen, PVOH sowie wenigstens einen mehrwertigen Alkohol aufweist. Besonders bevorzugt weist die wenigstens eine Gelphase mindestens ein Amylasegranulat und/oder mindestens eine Proteasegranulat, PVOH und wenigstens einen mehrwertigen Alkohol auf.

Erfindungsgemäß umfasst die wenigstens eine Gelphase mindestens ein Enzym, bevorzugt Enzymgranulat, bevorzugt mindestens ein Amylasegranulat und/oder mindestens eine Proteasegranulat, insbesondere mindestens ein Proteasegranulat, PVOH und/oder dessen Derivate in einem Anteil von etwa 4 Gew.-% bis 40 Gew.-%, insbesondere von 6 Gew.-% bis 30 Gew.-%, bevorzugt von 7 bis 24 Gew.-%, insbesondere bevorzugt zwischen 8 Gew.-% bis 22 Gew.-%. Deutlich geringere Anteile an PVOH, führen nicht zur Ausbildung einer stabilen Gelphase. Die Werte sind jeweils bezogen auf das Gesamtgewicht der Gelphase.

Gemäß einer ganz besonders bevorzugten Ausführungsform umfasst die wenigstens eine Gelphase PVOH (Polyvinylalkohol). Diese somit hergestellten Gelphasen sind besonders hochschmelzend, formstabil (auch bei 40 °C) und verändern ihre Form auch bei Lagerung nicht oder nur unwesentlich. Insbesondere sind sie auch wenig reaktiv im Hinblick auf eine direkte negative Wechselwirkung mit Bestandteilen des körnigen Gemenges, insbesondere der Pulverphase. PVOH kann insbesondere auch ohne Schwierigkeiten wasserarme bzw. wasserfreie Gelphasen erzeugen. Bei der Verwendung von PVOH als Polymer für die wenigstens eine Gelphase ergeben sich bei 110-120 °C dünnflüssige Schmelzen, die dadurch besonders leicht verarbeitet werden können, insbesondere kann das Einfüllen der Gelphase in die wasserlösliche Umhüllung schnell und genau vorgenommen werden, ohne dass ein Verkleben stattfindet oder die Menge ungenau dosiert wird. Des Weiteren haften diese Gelphasen besonders gut an der wasserlöslichen Umhüllung, insbesondere, wenn diese aus ebenfalls aus PVOH hergestellt ist. Dies ist auch optisch von Vorteil. Durch die schnelle Verfestigung der wenigstens einen Gelphase mit PVOH kann die Weiterverarbeitung der Gelphasen besonders schnell erfolgen. Weiterhin ist die gute Löslichkeit der erzeugten Gelphasen für die Gesamtlöslichkeit des Reinigungsmittels besonders günstig. Außerdem sind Gelphasen mit solch kurzen Verfestigungszeiten vorteilhaft, damit die darauf dosierte wenigstens eine feste Phase, umfassend körnige Gemenge, insbesondere Pulver, nicht in das noch nicht völlig erstarrte oder zu weiche Gel einsinkt. Dies führt optisch zu wenig ansprechenden Reinigungsmittelportionen.

Insbesondere bei den erfindungsgemäßen mehrphasigen Einmalportionen mit mindestens einer festen Phase ist es wichtig, dass die wenigstens eine Gelphase formstabil ist, damit möglichst wenig Interaktionen zwischen der festen und der Gelphase stattfinden können. Umfasst die wenigstens eine Gelphase neben PVOH weiterhin Gelatine, wird die Zähigkeit der Gelphase in der Herstellung erhöht.

Ein weiterer bevorzugter Gegenstand der vorliegenden Erfindung sind Reinigungsmittel, bevorzugt Geschirrspülmittel, insbesondere maschinelle Geschirrspülmittel, die in der gelförmigen Phase mindestens ein organisches Lösungsmittel, insbesondere ausgewählt ist aus 1,2-Propandiol, 1,3-Propandiol, Glycerin, 1,1,1-Trimethylolpropan, Triethylenglykol, Dipropylenglykol, Polyethlenglykole und/oder Mischungen daraus, enthalten.

Die wenigstens eine Gelphase umfasst bevorzugt wenigstens einen mehrwertigen Alkohol. Der wenigstens eine mehrwertige Alkohol ermöglicht neben der Herstellung von fließfähigen Gelphasen auch die Herstellung einer formstabilen, nicht-fließfähigen Gelphase innerhalb einer kurzen Erstarrungszeit, die innerhalb von 15 min oder weniger, insbesondere von 10 min oder weniger. Mehrwertige Alkohole im Sinne der vorliegenden Erfindung sind Kohlenwasserstoffe, in denen zwei, drei oder mehr Wasserstoffatome durch OH-Gruppen ersetzt sind. Dabei sind die OH-Gruppen an jeweils verschiedenen Kohlenstoffatomen gebunden. Ein Kohlenstoffatom weist keine zwei OH-Gruppen auf. Dies steht im Unterschied zu (einfachen) Alkoholen, bei welchen in Kohlenwasserstoffen nur ein Wasserstoffatom durch eine OH-Gruppe ersetzt ist. Mehrwertige Alkohole mit zwei OH-Gruppen werden als Alkandiole bezeichnet, mehrwertige Alkohole mit drei OH-Gruppen als Alkantriole. Ein mehrwertiger Alkohol entspricht damit der allgemeinen Formel [KW](OH)ₓ, wobei KW für einen Kohlenwasserstoff steht, der linear oder verzweigt, gesättigt oder ungesättigt, substituiert oder unsubstituiert ist. Eine Substituierung kann beispielweise mit-SH oder -NH- Gruppen erfolgen. Bevorzugt ist KW ein linearer oder verzweigter, gesättigter oder ungesättigter, unsubstituierter Kohlenwasserstoff. KW umfasst dabei wenigstens zwei Kohlenstoffatome. Der mehrwertige Alkohol umfasst 2, 3 oder mehr OH-Gruppen (x= 2, 3, 4 ...), wobei an jedem C-Atom des KW lediglich eine OH-Gruppe gebunden ist. Besonders bevorzugt umfasst KW 2 bis 10, also 2, 3, 4, 5, 6, 7, 8, 9, oder 10 Kohlenstoffatome. Eingesetzt werden können insbesondere mehrwertige Alkohole mit x=2, 3 oder 4 (beispielsweise z.B. Pentaerythrit mit x=4). Bevorzugt ist x=2 (Alkandiol) und/oder x=3 (Alkantriol).

Besonders bevorzugt umfasst die wenigstens eine Gelphase wenigstens ein Alkantriol und/oder wenigstens ein Alkandiol, insbesondere wenigstens ein C₃- bis C₁₀-Alkantriol und/oder wenigstens ein C₃- bis C₁₀- Alkandiol, bevorzugt wenigstens ein C₃- bis C₈-Alkantriol und/oder wenigstens ein C₃- bis C₈- Alkandiol, besonders wenigstens ein C₃- bis C₆-Alkantriol und/oder wenigstens ein C₃-bis C₅- Alkandiol als mehrwertigen Alkohol. Bevorzugt umfasst sie ein Alkantriol und ein Alkandiol als wenigstens einen mehrwertigen Alkohol. In einer bevorzugten Ausführungsform umfasst die wenigstens Gelphase daher wenigstens ein Polymer, insbesondere PVOH oder PVOH mit Gelatine, sowie wenigstens ein Alkandiol und wenigstens ein Alkantriol, insbesondere ein Alkantriol und ein Alkandiol. Ebenso bevorzugt ist eine Gelphase, die wenigstens ein Polymer, PVOH oder PVOH mit Gelatine, sowie ein C₃- bis C₈-Alkandiol und ein C₃- bis C₈- Alkantriol umfasst. Weiter bevorzugt ist eine Gelphase, die wenigstens ein Polymer, insbesondere PVOH oder PVOH mit Gelatine, sowie ein C₃- bis C₅-Alkandiol und ein C₃- bis C₆- Alkantriol umfasst. Erfindungsgemäß umfassen die mehrwertigen Alkohole keine Derivate, wie Ether, Ester etc. hiervon.

Überraschenderweise hat sich gezeigt, dass bei der Kombination eines entsprechenden Triols (Alkantriols) mit einem entsprechenden Diol (Alkandiol) besonders kurze Erstarrungszeit erreicht werden können. Die erhaltenen Gelphasen sind zudem transparent und weisen eine glänzende Oberfläche auf, die für einen ansprechenden optischen Eindruck des erfindungsgemäßen Reinigungsmittels sorgen. Die Begriffe Diol und Alkandiol werden vorliegend synonym verwendet. Gleiches gilt für Triol und Alkantriol.

Gemäß einer besonders bevorzugten Ausführungsform ist in den erfindungsgemäßen Reinigungsmitteln, bevorzugt Geschirrspülmitteln, insbesondere maschinellen Geschirrspülmitteln das mindestens eine organische Lösungsmittel in der gelförmigen Phase in Mengen von 30 bis 90 Gew.-%, insbesondere von 40 bis 85 Gew.-%, besonders bevorzugt von 50 bis 80 Gew.-%, bezogen auf das Gesamtgewicht der gelförmigen Phase, enthalten.

Die Menge an in erfindungsgemäßen Gelphasen eingesetzten mehrwertigen Alkohol oder mehrwertigen Alkoholen liegt vorzugsweise bei wenigstens 45 Gew.-%, insbesondere bei 55 Gew.-% oder mehr. Bevorzugte Mengenbereiche sind hierbei von 45 Gew.-% bis 85 Gew.-%, insbesondere von 50 Gew.-% bis 80 Gew.-%, bezogen auf das Gesamtgewicht der Gelphase.

Bevorzugt ist das C₃- bis C₆-Alkantriol Glyzerin und/oder 2-Ethyl-2-(hydroxymethyl)-1,3-propandiol (auch 1,1,1-Trimethylolpropan genannt) und/oder 2-Amino-2-(hydroxymethyl)-1,3-propandiol (TRIS, Trishydroxymethylaminoethan) und/oder 1,3,5-Pentantriol.

Besonders bevorzugt ist das C₃- bis C₆-Alkantriol Glyzerin und/oder 2-Ethyl-2-(hydroxymethyl)-1,3-propandiol (auch 1,1,1-Trimethylolpropan genannt). Das C₃- bis C₅- Alkandiol ist beispielsweise 1,5-Pentandiol, 3-Methyl- 1,5-Pentandiol, 1,4-Butandiol, 1,3-Propandiol und/oder 1,2-Propandiol, vorzugsweise 1,3-Propandiol und/oder 1,2-Propandiol. Überraschenderweise hat sich gezeigt, dass die Kettenlänge des Diols sowie insbesondere die Stellung der OH-Gruppen Einfluss auf die Transparenz der Gelphase hat. Vorzugsweise sind daher die OH-Gruppen des Diols nicht an unmittelbar benachbarten C-Atomen angeordnet. Insbesondere befinden sich zwischen den beiden OH-Gruppen des Diols drei oder vier Kohlenstoffatome, insbesondere 3 Kohlenstoffatome. Besonders bevorzugt ist das Diol 1,3-Propandiol. Überraschenderweise hat sich gezeigt, dass mit Mischungen, welche Glyzerin und 1,3-Propandiol und/oder 1,2-Propandiol umfassen, besonders gute Ergebnisse erzielt werden.

Erfindungsgemäß werden bevorzugt zusätzlich Polyethylenglykol(e) mit einer mittleren Molmasse von 200 bis 600 g/mol in der wenigstens einen Gelphase bzw. den Gelphasen eingesetzt. Dabei werden in Kombination mit Polyvinylalkohol Polyethylenglykole mit einer mittleren Molmasse zwischen etwa 200 und etwa 600 g/mol,bevorzugt zwischen 300 und 500 g/mol, insbesondere bevorzugt zwischen 350 und 450 g/mol, beispielsweise um 400 g/mol INCI: PEG400) eingesetzt. Erfindungsgemäße Reinigungsmittelportion sind somit dadurch gekennzeichnet, dass sie Polyethylenglykol(e) mit einer mittleren Molmasse von 300 bis 500 g/mol, insbesondere von 350 bis 450 g/mol aufweisen.

Erfindungsgemäß umfasst die wenigstens eine Gelphase mindestens ein Enzym, bevorzugt Enzymgranulat, bevorzugt mindestens ein Amylasegranulat und/oder mindestens eine Proteasegranulat, insbesondere mindestens ein Proteasegranulat, PVOH und/oder dessen Derivate, bevorzugt in einem Anteil von etwa 4 Gew.-% bis 40 Gew.-%, insbesondere von 6 Gew.-% bis 30 Gew.-%, bevorzugt von 7 bis 24 Gew.-%, insbesondere bevorzugt zwischen 8 Gew.-% bis 22 Gew.-% und mindestens einen mehrwertigen Alkohol ggf. zusätzlich Polyethylenglykole mit einer mittleren Molmasse von etwa 200 bis 600 g/mol in Mengen von 5 bis 30 Gew.-%, bevorzugt von 8 bis 26 Gew.-%, insbesondere von 10 bis 22 Gew.-% bezogen auf das Gesamtgewicht der wenigstens einen Gelphase enthalten.

Überraschenderweise hat sich gezeigt, dass die Zugabe von Polyethylenglykolen, insbesondere von solchen mit mittleren Molmassen von 200 bis zu 600 g/mol zu der wenigstens einen Gelphase, insbesondere bei Gelphasen, umfassend Polyvinylalkohol, zu einer Beschleunigung der Verfestigungszeit der Gelphasen führt. Es können dabei Werte von wenigen Minuten und sogar unter einer Minute erreicht werden. Dies ist insbesondere für die produktionstechnischen Abläufe von großem Vorteil, da die Weiterverarbeitung der Gelphasen im verfestigten Zustand viel schneller und damit in der Regel kostengünstiger erfolgen kann. Überraschenderweise wurde gefunden, dass die Anwesenheit von Polyethylenglykol(en) mit einer mittleren Molmasse von 200 bis 600 g/mol in Kombination mit Polyvinylalkohol und/oder dessen Derivaten entscheidend dazu beiträgt, die Erstarrungszeiten zu verringern. Ohne auf die Theorie festgelegt zu sein, wird davon ausgegangen, dass solche Polyethylenglykole, insbesondere solche mit einer Molmasse von 350 bis 450 g/mol, insbesondere um 400 g/mol die Sol-Gel-Temperatur erhöht.

In einer besonders bevorzugten Ausführungsform beträgt die Menge von Polyethylenglykol(en) mit einer mittleren Molmasse von 350 bis 450 g/mol, beispielsweise um 400 g/mol, 10 bis 22 Gew.-% bezogen auf das Gesamtgewicht der Gelphase.

Erfindungsgemäß umfasst die wenigstens eine Gelphase mindestens ein Enzym, bevorzugt Enzymgranulat, bevorzugt mindestens ein Amylasegranulat und/oder mindestens eine Proteasegranulat, insbesondere mindestens ein Proteasegranulat, PVOH und/oder dessen Derivate in einem Anteil von etwa 4 Gew.-% bis 40 Gew.-%, insbesondere von 6 Gew.-% bis 30 Gew.-%, bevorzugt von 7 bis 24 Gew.-%, insbesondere bevorzugt zwischen 8 Gew.-% bis 22 Gew.-%, Polyethylenglykol(e) mit einer mittleren Molmasse von 200 bis 600 g/mol und 1,3-Propandiol und Glyzerin bzw. 1,1,1-Trimethylolpropan als mehrwertige Alkohole. Eine besonders bevorzugt Gelphase umfasst Gelatine oder PVOH als Polymer und 1,3-Propandiol und Glyzerin bzw. 1,1,1-Trimethylolpropan als mehrwertige Alkohole. Umfasst die Gelphase jeweils bezogen auf das Gesamtgewicht der gelförmigen Phase ein Alkantriol, insbesondere Glyzerin oder 1,1,1-Trimethylolpropan, so beträgt der Anteil an Alkantriol, insbesondere Glyzerin oder 1,1,1-Trimethylolpropan, bezogen auf das Gesamtgewicht der Gelphase, zwischen 3 und 75 Gew.-%, bevorzugt 5 Gew.-% bis 70 Gew.-%, insbesondere 10 Gew.-% bis 65 Gew.-%, besonders 20 Gew.-% bis 40 Gew.-%.

Umfasst die Gelphase ggf. mehrere Alkantriol(e), so beträgt der Gesamtanteil an Alkantriol(en), bezogen auf das Gesamtgewicht der Gelphase, zwischen 3 und 75 Gew.-%, bevorzugt 5 Gew.-% bis 70 Gew.-%, insbesondere 10 Gew.-% bis 65 Gew.-%, besonders 20 Gew.-% bis 40 Gew.-%.

Ist Glyzerin als Alkantriol in der Gelphase enthalten, so beträgt der Anteil an Glyzerin bezogen auf das Gesamtgewicht der Gelphase, bevorzugt 5 Gew.-% bis 70 Gew.-%, insbesondere 10 Gew.-% bis 65 Gew.-%, besonders 20 Gew.-% bis 40 Gew.-%.

Ist 1,1,1-Trimethylolpropan in der Gelphase enthalten, so beträgt der Anteil an 1,1,1-Trimethylolpropan bezogen auf das Gesamtgewicht der Gelphase, bevorzugt 5 Gew.-% bis 70 Gew.-%, insbesondere 10 Gew.-% bis 65 Gew.-%, besonders bevorzugt 18 bis 45 Gew.-%, insbesondere bevorzugt 20 Gew.-% bis 40 Gew.-%.

Ist 2-Amino-2-Hydroxymethyl-1,3-propandiol in der Gelphase enthalten, so beträgt der Anteil an 2-Amino-2-Hydroxymethyl-1,3-propandiol, bezogen auf das Gesamtgewicht der Gelphase, bevorzugt 5 Gew.-% bis 70 Gew.-%, insbesondere 10 Gew.-% bis 65 Gew.-%, besonders 20 Gew.-% bis 40 Gew.-%.

Sind ggf. mehrere Alkandiole in der Gelphase enthalten, beträgt der Anteil an Alkandiolen, bezogen auf das Gesamtgewicht der Gelphase, bevorzugt 5 Gew.-% bis 70 Gew.-%, insbesondere 7 Gew.-% bis 65 Gew.-%, besonders 10 Gew.-% bis 40 Gew.-%.

Umfasst die Gelphase jeweils bezogen auf das Gesamtgewicht der gelförmigen Phase neben mindestens ein Enzym, bevorzugt Enzymgranulat, bevorzugt mindestens einem Amylasegranulat und/oder mindestens einem Proteasegranulat, insbesondere mindestens ein Subtilisin-haltiges Proteasegranulat, mindestens mindestens ein Alkandiol, insbesondere 1,3-Propandiol oder 1,2-Propandiol, so beträgt der Anteil an Alkandiol, insbesondere 1,3-Propandiol oder 1,2-Propandiol, bezogen auf das Gesamtgewicht der Gelphase, bevorzugt 5 Gew.-% bis 70 Gew.-%, insbesondere 10 Gew.-% bis 65 Gew.-%, besonders 20 Gew.-% bis 45 Gew.-%. Ist 1,3-Propandiol in der Gelphase enthalten, so beträgt der Anteil an 1,3-Propandiol, bezogen auf das Gesamtgewicht der Gelphase, insbesondere 10 Gew.-% bis 65 Gew.-%, besonders 20 Gew.-% bis 45 Gew.-%.

Bevorzugt ist eine Gelphase, die jeweils bezogen auf das Gesamtgewicht der gelförmigen Phase neben mindestens einem Enzym, bevorzugt Enzymgranulat, bevorzugt mindestens einem Amylasegranulat und/oder mindestens einem Proteasegranulat, insbesondere mindestens einem Subtilisin-haltiges Proteasegranulat, 20 bis 45 Gew.-% 1,3 Propandiol und/oder 1,2-Propandiol und 10 Gew.-% bis 65 Gew.-% 2-Amino-2-Hydroxymethyl-1,3-propandiol, jeweils bezogen auf das Gesamtgewicht der Gelphase, enthält. Ebenfalls bevorzugt ist eine Gelphase, die 20 bis 45 Gew.-% 1,3 Propandiol und/oder 1,2-Propandiol und 10 Gew.-% bis 65 Gew.-% 1,1,1-Trimethylolpropan, jeweils bezogen auf das Gesamtgewicht der Gelphase, enthält. Insbesondere bevorzugt ist eine Gelphase, die 20 bis 45 Gew.-% 1,3 Propandiol und/oder 1,2-Propandiol und 10 Gew.-% bis 65 Gew.-% Glyzerin, jeweils bezogen auf das Gesamtgewicht der Gelphase, enthält. Es hat sich gezeigt, dass in diesen Bereichen eine rasche Erstarrung bei 20 °C einer Gelphase möglich ist, die erhaltenen Phasen lagerstabil und transparent sind. Insbesondere der Anteil an Glyzerin hat eine Auswirkung auf die Aushärtezeit.

Weist die erfindungsgemäße wenigstens eine Gelphase jeweils bezogen auf das Gesamtgewicht der gelförmigen Phase neben mindestens einem Enzym, bevorzugt Enzymgranulat, bevorzugt mindestens einem Amylasegranulat und/oder mindestens einem Subtilisin-haltiges Proteasegranulat, insbesondere mindestens einem Subtilisin-haltiges Proteasegranulat, ein C₃- bis C₆-Alkantriol und ein C₃- bis C₅- Alkandiol auf, so beträgt deren Gewichtsverhältnis vorzugsweise 3:1 bis 1:2. Insbesondere beträgt das Gewichtsverhältnis von 2:1 bis 1:1,5, bevorzugt von 1,5:1 bis 1:1,2, bevorzugt von 1,3 bis 1:1, wenn Glyzerin und 1,3-Propandiol als mehrwertige Alkohole enthalten sind. Überraschenderweise hat sich gezeigt, dass bei diesen Gewichtsverhältnissen innerhalb kurzer Erstarrungszeiten bei 20° C von 10 Minuten oder weniger, lagerstabile, glänzende, transparente Gelphasen erhalten werden können. In Kombination mit Polyethylenglykolen mit einer mittleren Molmasse von 200 bis 600 g/mol, lässt sich bei den bevorzugt genannten Gewichtsverhältnissen, insbesondere Gewichtsverhältnissen (C₃- bis C₆-Alkantriol : C₃- bis C₅-Alkandiol) von 1,5:1 bis 1:1,2, auf Erstarrungszeiten von 5 Minuten und weniger senken.

Gemäß einer weiteren bevorzugten Ausführungsform kann zusätzlich zu den vorstehend genannten Alkanolen Triethylenglykol in der wenigstens einen Gelphase, insbesondere die vorstehend als bevorzugt beschriebenen Gelphasen enthalten sein, insbesondere, wenn diese Phase PVOH und ggf. Polyethylenglykole mit einer mittleren Molmasse von 200 bis 600 g/mol enthält. Triethylenglykol beschleunigt dabei vorteilhafterweise die Verfestigung der Gelphase(n). Außerdem führt es dazu, dass die resultierende Gelphase, wenn überhaupt, nur geringfügig, nicht beobachtbar Flüssigkeit mit der Umgebung austauscht. Dies verbessert insbesondere den optischen Eindruck der resultierenden Reinigungsmittelportionen. Besonders bevorzugt ist dabei, wenn die wenigstens eine Gelphase, jeweils bezogen auf das Gesamtgewicht der gelförmigen Phase neben mindestens einem Enzym, bevorzugt Enzymgranulat, bevorzugt mindestens einem Amylasegranulat und/oder mindestens einem Proteasegranulat, insbesondere mindestens einem Subtilisin-haltiges Proteasegranulat,1,3- und/oder 1,2-Propandiol, besonders bevorzugt 1 bis 3,5 Gew.-% 1,3-Propandiol, und Glyzerin zwischen 0,1 und 20 Gew.-%, bevorzugt zwischen 1 und 15 Gew.-%, insbesondere zwischen 5 und 12 Gew.-%, beispielsweise 8 bis 11 Gew.-% Triethylenglykol enthält

Weiterhin bevorzugt umfasst die wenigstens eine Gelphase ein weiteres anionisches Polymer, insbesondere Polycarboxylate. Diese können entweder als Gerüststoffe und/oder als Verdickungspolymer wirken. Erfindungsgemäß kann die wenigstens eine Gelphase weiterhin anionische Polymere oder Copolymere mit Gerüststoffeigenschaften umfassen. Bevorzugt handelt es sich hierbei um ein Polycarboxylat. Als Polycarboxylat wird vorzugsweise ein copolymeres Polyacrylat, vorzugsweise ein Sulfopolymer vorzugsweise ein copolymeres Polysulfonat, vorzugsweise ein hydrophob modifiziertes copolymeres Polysulfonat eingesetzt. Die Copolymere können zwei, drei, vier oder mehr unterschiedliche Monomereinheiten aufweisen. Bevorzugte copolymere Polysulfonate enthalten neben Sulfonsäuregruppen-haltigem(n) Monomer(en) wenigstens ein Monomer aus der Gruppe der ungesättigten Carbonsäuren.

Gemäß einer besonders bevorzugten Ausführungsform enthält die wasserarme gelförmige Phase ein Polymer umfassend mindestens ein sulfonsäuregruppenhaltige Monomer.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH2, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Bei den Sulfonsäuregruppen-haltigen Monomeren sind solche der Formel R⁵(R⁶)C=C(R⁷)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit - NH₂, -OH oder -COOH substituierte Alkyl- oder Alkenylreste oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, - C(O)-NH-C(CH₃)₂-CH₂- und -C(O)-NH-CH(CH₃)-CH₂-.

Unter diesen Monomeren bevorzugt sind solche der Formeln H₂C=CH-X-SO₃H, H₂C=C(CH₃)-X-SO₃H oder HO₃S-X-(R⁶)C=C(R⁷)-X-SO₃H, in denen R⁶ und R⁷ unabhängig voneinander ausgewählt sind aus -H, -CH₃, -CH₂CH₃, - CH₂CH₂CH₃ und -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, - C(O)-NH-C(CH₃)₂-CH₂- und -C(O)-NH-CH(CH₃)-CH₂-.

Gemäß einer besonders bevorzugten Ausführungsform enthält die gelförmige Phase ein Polymer umfassend als sulfonsäuregruppenhaltiges Monomer Acrylamidopropansulfonsäuren, Methacrylamidomethylpropansulfonsäuren oder Acrylamidomethylpropansulfonsäure.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze. In den Polymeren können die Sulfonsäuregruppen ganz oder teilweise in neutralisierter Form vorliegen, das heißt dass das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Der Einsatz von teil- oder vollneutralisierten Sulfonsäuregruppen-haltigen Copolymeren ist erfindungsgemäß bevorzugt.

Die Monomerenverteilung der erfindungsgemäß bevorzugt eingesetzten Copolymere beträgt bei Copolymeren, die nur Carbonsäuregruppen-haltige Monomere und Sulfonsäuregruppen-haltige Monomere enthalten, vorzugsweise jeweils 5 bis 95 Gew.-%, besonders bevorzugt beträgt der Anteil des Sulfonsäuregruppen-haltigen Monomers 50 bis 90 Gew.-% und der Anteil des Carbonsäuregruppen-haltigen Monomers 10 bis 50 Gew.-%, die Monomere sind hierbei vorzugsweise ausgewählt aus den zuvor genannten. Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte Reinigungsmittel sind dadurch gekennzeichnet, dass die Copolymere Molmassen von 2000 bis 200.000 g-mol-¹, vorzugsweise von 4000 bis 25.000 g.mol⁻¹ und insbesondere von 5000 bis 15.000 g.mol⁻¹ aufweisen.

In einer weiteren bevorzugten Ausführungsform umfassen die Copolymere neben Carboxylgruppen-haltigem Monomer und Sulfonsäuregruppen-haltigem Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer Geschirrspülmittel verbessert werden.

Besonders bevorzugt umfasst die wenigstens eine Gelphase weiterhin ein anionisches Copolymer, wobei als anionisches Copolymer ein Copolymer, umfassend
i) Carbonsäuregruppen-haltige Monomere
ii) Sulfonsäuregruppen-haltige Monomere
iii) nichtionische Monomere, insbesondere hydrophobe Monomere eingesetzt wird.

Als nichtionische Monomere werden vorzugsweise Monomere der allgemeinen Formel R¹(R²)C=C(R³)-X-R⁴ eingesetzt, in der R¹ bis R³ unabhängig voneinander für -H, -CH₃ oder - C₂H₅ steht, X für eine optional vorhandene Spacergruppe steht, die ausgewählt ist aus -CH₂-, - C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2,2,3-Dimethylhexen-1, 2,4-Diemthylhexen-1, 2,5-Dimethlyhexen-1, 3,5-Dimethylhexen-1, 4,4-Dimethylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C₂₂-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2-Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, *N*-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, *N*-(Lauryl)acrylamid, Acrylsäurestearylester, Methacrylsäurestearylester, *N*-(Stearyl)acrylamid, Acrylsäurebehenylester, Methacrylsäurebehenylester und N-(Behenyl)acrylamid oder deren Mischungen, insbesondere Acrylsäure, Ethylacrylat, 2-Acrylamido-2-methylpropansulfonsäure (AMPS) sowie deren Mischungen.

Überaschenderweise hat sich gezeigt, dass auch PVOH und/oder dessen Derivate zusammen mit anionischen Polymeren oder Copolymeren, insbesondere mit Sulfonsäuregruppenhaltigen Copolymeren, zur Ausbildung von Gelphasen mit unempfindlichen Oberflächen führt. Entsprechende Oberflächen können vom Endverbraucher berührt werden, ohne dass Material an den Händen haften bleibt. Auch in einer Verpackung findet kein Materialabtrag statt. Bevorzugt umfasst daher die Gelphase somit PVOH, Polyethylenglykol(e) mit einer mittleren Molmasse von 200 bis 600 g/mol, mindestens einen mehrwertigen Alkohol und ein anionisches Copolymer/Polymer. Der Anteil am anionischen Polymer beträgt vorzugsweise 1 Gew.-% bis 35 Gew.-%, insbesondere 3 Gew.-% bis 30 Gew.-%, besonders 4 Gew.-% bis 25 Gew.-%, bevorzugt 5 Gew.-% bis 20 Gew.-%, beispielsweise 10 Gew.-% bezogen auf das Gesamtgewicht der Gelphase. Sulfopolymere, insbesondere die bevorzugten copolymeren Polysulfonate, welche neben Sulfonsäuregruppen-haltigem(n) Monomer(en) wenigstens ein Monomer aus der Gruppe der ungesättigten Carbonsäuren, insbesondere Acrylsäure enthalten, sorgen zudem für einen hervorragenden Glanz der Oberfläche. Zudem bleiben auch Fingerabdrücke nicht erhalten. Daher beträgt der Anteil an Sulfopolymeren, insbesondere die bevorzugten copolymeren Polysulfonate, welche neben Sulfonsäuregruppen-haltigem(n) Monomer(en) wenigstens ein Monomer aus der Gruppe der ungesättigten Carbonsäuren, insbesondere Acrylsäure enthalten, insbesondere an den genannten Sulfopolymeren mit AMPS als sulfonsäuregruppenhaltigem Monomer, beispielsweise Acusol 590, Acusol 588 oder Sokalan CP50, vorzugsweise 1 Gew.-% bis 25 Gew.-%, insbesondere 3 Gew.-% bis 18 Gew.-%, besonders 4 Gew.-% bis 15 Gew.-%, bevorzugt 5 Gew.-% bis 12 Gew.-% bezogen auf das Gewicht der Gelphase. In einer besonders bevorzugten Ausführungsform umfasst die wenigstens eine Gelphase neben mindestens einem Enzym, bevorzugt Enzymgranulat, bevorzugt mindestens einem Amylasegranulat und/oder mindestens einem Proteasegranulat, insbesondere mindestens einem Subtilisin-haltiges Proteasegranulat, daher PVOH sowie ein Sulfopolymer, insbesondere die bevorzugten copolymeren Polysulfonate, welche neben Sulfonsäuregruppen-haltigem(n) Monomer(en) wenigstens ein Monomer aus der Gruppe der ungesättigten Carbonsäuren, insbesondere Acrylsäure enthalten, und wenigstens einen mehrwertigen Alkohol.

Gemäß einer weiteren Ausführungsform können neben den genannten Polyethylenglykolen mit einer mittleren Molmasse von 200 bis 600 g/mol weitere Polyalkylenglykolen, insbesondere weitere Polyethylenglykole, mit einer mittleren Molmasse zwischen etwa 800 und 8000 in der wenigstens einen Gelphase enthalten sein. Besonders bevorzugt werden die vorstehend genannten Polyethylenglykole in Mengen von 1 bis 40 Gew.-%, bevorzugt 5 bis 35 Gew.-%, insbesondere von 10 bis 30 Gew.-%, beispielsweise 15 bis 25 bevorzugt jeweils bezogen auf das Gesamtgewicht der Gelphase eingesetzt.

Ganz besonders bevorzugte Ausführungsformen der vorliegenden Erfindung umfassen als wenigstens eine Gelphase jeweils bezogen auf das Gesamtgewicht der gelförmigen Phase neben mindestens einem Enzym, bevorzugt Enzymgranulat, bevorzugt mindestens einem Amylasegranulat und/oder mindestens einem Proteasegranulat, insbesondere mindestens einem Subtilisin-haltiges Proteasegranulat, 8 bis 22 Gew.-% PVOH, 15 bis 40 Gew.-% 1,3-Propandiol, 20 bis 40 Gew.-% Glyzerin, 5 bis 15 Gew.-% Sulfonsäuregruppenhaltiges Polyacrylat-Copolymer, und 8 bis 22 Gew.-%, insbesondere 10 bis 20 Gew.-%, Polyethylenglykol mit einer mittleren Molmasse von 200-600 g/mol, optional 2 bis 10 Gew.-% 1,2-Propandiol, sowie optional zusätzlich auch 2- 15 Gew.-% Triethylenglykol jeweils bezogen auf das Gesamtgewicht der Gelphase.

Gemäß einer weiteren besonders bevorzugten Ausführungsform handelt es sich bei dem Reinigungsmittel, bevorzugt Geschirrspülmittel, insbesondere maschinellem Geschirrspülmittel um eine Reinigungsmittelportion in einer wasserlöslichen Umhüllung mit einer oder mehreren Kammern/Kompartimenten. Dabei ist das Reinigungsmittel bevorzugt als Reinigungsmitteleinmalportion konfektioniert, so dass es zur Durchführung eines Geschirrspülmaschinendurchlaufs eingesetzt wird und dabei (weitestgehend) im wesentlichen verbraucht wird.

Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf.

Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10.000 bis 1.000.000 gmol⁻¹, vorzugsweise von 20.000 bis 500.000 gmol⁻¹, besonders bevorzugt von 30.000 bis 100.000 gmol⁻¹ und insbesondere von 40.000 bis 80.000 gmol⁻¹ liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

In einer bevorzugten Ausführungsform besteht die wasserlösliche Verpackung zu mindestens 20 Gew.-%, besonders bevorzugt zu mindestens 40 Gew.-%, ganz besonders bevorzugt zu mindestens 60 Gew.-% und insbesondere zu mindestens 80 Gew.-% aus einem Polyvinylalkohol, dessen Hydrolysegrad 70 bis 100 Mol-%, vorzugsweise 80 bis 90 Mol-%, besonders bevorzugt 81 bis 89 Mol-% und insbesondere 82 bis 88 Mol-%, beträgt.

Einem zur Herstellung der wasserlöslichen Umhüllung geeignetem Polyvinylalkohol-enthaltendem Folienmaterial kann zusätzlich ein Polymer ausgewählt aus der Gruppe umfassend (Meth)Acrylsäure-haltige (Co)Polymere, Polyacrylamide, Oxazolin-Polymere, Polystyrolsulfonate, Polyurethane, Polyester, Polyether, Polymilchsäure oder Mischungen der vorstehenden Polymere zugesetzt sein. Ein bevorzugtes zusätzliches Polymer sind Polymilchsäuren.

Bevorzugte Polyvinylalkoholcopolymere umfassen neben Vinylalkohol Dicarbonsäuren als weitere Monomere. Geeignete Dicarbonsäure sind Itaconsäure, Malonsäure, Bernsteinsäure und Mischungen daraus, wobei Itaconsäure bevorzugt ist. Ebenfalls bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Es kann bevorzugt sein, dass das Folienmaterial weitere Zusatzstoffe enthält. Das Folienmaterial kann beispielsweise Weichmacher wie Dipropylenglycol, Ethylenglycol, Diethylenglycol, Propylenglycol, Glyzerin, Sorbitol, Mannitol oder Mischungen daraus enthalten. Weitere Zusatzstoffe umfassen beispielsweise Freisetzungshilfen, Füllmittel, Vernetzungsmittel, Tenside, Antioxidationsmittel, UV-Absorber, Antiblockmittel, Antiklebemittel oder Mischungen daraus.

Geeignete wasserlösliche Folien zum Einsatz in den wasserlöslichen Umhüllungen der wasserlöslichen Verpackungen gemäß der Erfindung sind Folien, die von der Firma MonoSol LLC beispielsweise unter der Bezeichnung M8720, M8630, M8312, M8440, M7062, C8400 oder M8900 vertrieben werden. Weiter geeignet sind Folien, welche unter der Bezeichnung SH2601, SH2504, SH2707 oder SH2701 von Nippon Gohsei vertrieben werden. Andere geeignete Folien umfassen Folien mit der Bezeichnung Solublon® PT, Solublon® GA, Solublon® KC oder Solublon® KL von der Aicello Chemical Europe GmbH oder die Folien VF-HP von Kuraray.

Die wasserlösliche Umhüllung weist bevorzugt zumindest teilweise einen Bitterstoff mit einem Bitterwert zwischen 1.000 und 200.000, insbesondere solche ausgewählt aus Chininsulfat (Bitterwert = 10.000), Naringin (Bitterwert = 10.000), Saccharoseoctaacetat (Bitterwert = 100.000), Chininhydrochlorid und Mischungen daraus auf. Insbesondere ist die äußere Oberfläche der wasserlöslichen Umhüllung zumindest teilweise mit einem Bitterstoff mit einem Bitterwert zwischen 1.000 und 200.000 beschichtet. In diesem Zusammenhang ist es insbesondere zu bevorzugen, dass die wasserlösliche Umhüllung zu mindestens 50 %, vorzugsweise zu mindestens 75 % und ganz besonders bevorzugt mindestens 90 % mit dem Bitterstoff mit einem Bitterwert zwischen 1.000 und 200.000 beschichtet ist. Das Aufbringen des Bitterstoffs mit einem Bitterwert zwischen 1.000 und 200.000 kann beispielsweise mittels Bedruckung, Besprühens oder Bestreichung erfolgen.

Erfindungsgemäß weist die wasserlösliche Umhüllung mindestens eine kontinuierlich umlaufende Siegelnaht auf, die im Wesentlichen in einer Ebene liegt. Dies ist prozesstechnisch günstig, da für eine Umlaufende Siegelnaht, welche im Wesentlichen in einer Ebene liegt, nur ein einziger Versiegelschritt, ggf. unter Benutzung nur eines einzigen Siegelwerkzeug, notwendig ist. Die kontinuierlich umlaufende Siegelnaht führt zu einem besseren Verschluss gegenüber solchen Umhüllungen mit mehreren Siegelnähten und einer hervorragenden Dichtigkeit der Siegelnaht und damit der Umhüllung selbst. Austreten von Produkt aus der Umhüllung, z.B. auf die Oberfläche der Portion wäre nachteilig, da der Konsument dann mit dem Produkt in Kontakt käme. Genau dies soll aber bei einer Reinigungsmittelportion mit einer wasserlöslichen Umhüllung möglichst vermieden werden.

Die wasserlösliche Umhüllung kann bevorzugt aus mindestens 2 Verpackungsteilen hergestellt werden. Bevorzugt sind die mindestens zwei Verpackungsteile wasserlöslich, damit in der Geschirrspülmaschine keine Verpackungsteile zurückbleiben, die dann zu Problemen in der Geschirrspülmaschine führen können. Dabei ist es nicht notwendig, dass die mindestens zwei Verpackungsteile unterschiedlich sind. Sie können bevorzugt aus dem gleichen Material und auf die gleiche Art und Weise hergestellt sein. In einer bevorzugten Ausführungsform handelt es sich dabei um zwei Teile einer wasserlöslichen Folie, insbesondere um zwei Teile einer wasserlöslichen Folie gleicher Zusammensetzung.

In einer weiteren Ausführungsform können die mindestens zwei Verpackungsteile aus unterschiedlichem Material, z.B. aus unterschiedlichen Folien oder aus Material mit zwei unterschiedlichen Eigenschaften (z.B. warm- und kaltwasserlösliche Folie) hergestellt sein. In dieser Ausführungsform ist es bevorzugt, dass eine wasserlösliche Folie und ein anderes Verpackungsteil, welches durch Spritzguss hergestellt wurde, kombiniert werden.

Gemäß einer besonders bevorzugten Ausführungsform der vorliegenden Erfindung umfasst die wasserlösliche Umhüllung mindestens eine zumindest teilweise plastisch verformte Folie. Insbesondere kann diese plastische Verformung der Folie durch dem Fachmann bekannte Methoden wie Tiefziehen (mit und ohne Anlegen eines Vakuums), Blas- oder Stempelformen hergestellt werden. Insbesondere umfasst die wasserlösliche Umhüllung mindestens eine zumindest teilweise plastisch verformte Folie, die durch Tiefziehen hergestellt wurde.

Die wenigstens eine feste Phase und die wenigstens eine Gelphase können innerhalb der wasserlöslichen Umhüllung in beliebiger Kombination zueinander angeordnet sein. So kann eine feste Phase auf oder neben einer Gelphase angeordnet sein. In dieser Ausführungsform weist das erfindungsgemäße Reinigungsmittel eine feste Phase und eine Gelphase auf. Es ist auch denkbar, dass eine feste Phase von Gelphasen umgeben ist. Auch ein Einbetten einer Phase in eine andere ist erfindungsgemäß umfasst. In einer weiteren, besonders bevorzugten Anordnung liegt die Gelphase in gegossener Form, beispielsweise in Form eines Gelkerns vor, der von einer festen Phase umgeben ist. Es können auch 2 oder mehr voneinander getrennte Kavitäten vorhanden sein, die mit der wenigstens einen Gelphase gefüllt werden. In dieser Ausführungsform umfasst das Reinigungsmittel zwei Gelphasen, wobei die zwei Gelphasen unterschiedliche Zusammensetzungen aufweisen können.

Gemäß einer bevorzugten Ausführungsform sind 3, 4, 5 oder 6 oder mehr voneinander getrennte Kavitäten vorhanden, die mit einer oder mehreren der Gelphasen gefüllt werden. Bevorzugt umfassen solche Reinigungsmittel 3, 4, 5 oder 6 oder mehr Gelphasen, wobei diese Gelphase die gleiche oder auch unterschiedliche Zusammensetzungen aufweisen können.

Ein bevorzugter Gegenstand der vorliegenden Erfindung ist ein Reinigungsmittel, bevorzugt Geschirrspülmittel, insbesondere maschinelles Geschirrspülmittel, welches zusätzlich mindestens eine feste, insbesondere partikuläre Phase sowie optional mindestens eine weitere flüssige/gelförmige oder feste Phase umfasst.

"Fest" bedeutet in diesem Zusammenhang, dass die Zusammensetzung bei Standardbedingungen (Temperatur 25°C, Druck 1013 mbar) in fester Form vorliegt. Geeignete feste Phasen sind zum einen körnige Gemenge einer festförmigen Zusammensetzung, wie zum Beispiel Pulver und/oder Granulate, insbesondere pulverförmige Phasen. Erfindungsgemäß ebenfalls geeignet sind feste Zusammensetzungen/Phasen, welche eine gegenüber dem losen Pulver verstärkte Formstabilität aufweisen, beispielsweise Pulver- oder Granulatzubereitung, welche vor oder nach dem Einschließen in die Folie durch Kompression verdichtet wurden, z.B. durch Rückstellkräfte der Folie nach dem Tiefziehen oder auch direkt komprimierte Zusammensetzungen, wie Komprimate oder Tabletten. Diese mindestens eine feste Phase können in direktem Kontakt mit der gelförmigen Phase stehen. Erfindungsgemäß sind weiterhin Reinigungsmittelportionen, insbesondere Mehrkammerbeutel, bei denen die feste und die gelförmige Phase in räumlicher Nähe, aber getrennt voneinander vorliegen. Dabei können die beiden Kammern beispielsweise durch eine Folie, insbesondere eine wasserlösliche Folie, oder auch durch eine Siegelnaht (bevorzugt von einer Siegelnaht von 3 mm oder weniger) getrennt sein. Erfindungsgemäß sind daher übereinanderliegende als auch nebeneinander angeordnete Kammern eines Mehrkammerpouches. Weiterhin sind auch Gemische von Ein- oder Mehrkammerbeutel, welche eine erfindungsgemäße gelförmige Phase und davon getrennt mindestens eine feste Phase umfassen, die durch Anordnung, z.B. durch Faltung und Fixierung eines Pouches, oder durch Lagerung in einem Abstand von geringer als 3 mm in Kontakt kommen, beispielsweise in einem Verpackungsbeutel oder einer Vorrichtung zur portionierten Dosierung, erfindungsgemäß.

Als pulverförmige Phase im Sinne der vorliegenden Erfindung ist ein körniges Gemenge zu verstehen, welches aus einer Vielzahl an losen, festförmigen Partikeln gebildet wird, die wiederum sogenannte Körner umfassen. Erfindungsgemäß umfasst der Begriff pulverförmige Phase Pulver und/oder Granulate gemäß der folgenden Definition.

Ein Korn ist eine Bezeichnung für die partikulären Bestandteile von Pulvern (Körner sind die losen, festförmigen Partikel), Stäuben (Körner sind die losen festförmigen Partikel), Granulaten (lose, festförmige Partikel sind Agglomerate aus mehreren Körnern) und anderen körnigen Gemengen. Eine bevorzugte Ausführungsform des körnigen Gemenges der Zusammensetzung der festen Phase ist das Pulver und/oder das Granulat, wenn hier von "Pulver" oder "Granulat" gesprochen wird, ist ebenfalls umfasst, dass es sich dabei auch um Mischungen verschiedener Pulver oder verschiedener Granulate handelt. Entsprechend sind mit Pulver und Granulat auch Mischungen verschiedener Pulver mit verschiedenen Granulaten gemeint. Die besagten festförmigen Partikel des körnigen Gemenges weisen wiederum bevorzugt einen Partikeldurchmesser X_{50,3} (Volumenmittel) von 10 bis 1500 µm, weiter bevorzugt von 200 µm bis 1200 µm, besonders bevorzugt von 600 µm bis 1100 µm, auf. Diese Partikelgrößen können durch Siebung oder mittels eines Partikelgrößenanalysators Camsizer der Fa. Retsch bestimmt werden. Das als feste Phase dienende körnige Gemenge der festförmigen Zusammensetzung der vorliegenden Erfindung liegt bevorzugt in rieselfähiger Form (besonders bevorzugt als rieselfähiges Pulver und/oder rieselfähiges Granulat) vor. Das Mittel der erfindungsgemäßen Portion umfasst somit wenigstens eine feste Phase eines rieselfähigen, körnigen Gemenges einer festförmigen Zusammensetzung, insbesondere eines Pulvers sowie wenigstens eine, wie zuvor, definierte Gelphase.

Ein besonders bevorzugter Gegenstand der vorliegenden Erfindung sind Reinigungsmittel, insbesondere Reinigungsmittelportionen, bei denen die gelförmige Phase in direktem Kontakt, beispielsweise in einer Kammer, mit der mindestens einen festen Phase enthalten ist.

Des Weiteren ist es bevorzugt, dass die wenigstens eine feste Phase und die wenigstens eine Gelphase in einem unmittelbaren Kontakt miteinander stehen. In diesem Fall soll es keine negative Wechselwirkung zwischen der festen Phase und der Gelphase geben. Keine negative Wechselwirkung bedeutet hier beispielsweise, dass keine Inhaltsstoffe oder Lösungsmittel aus einer Phase in die andere übertreten oder dass die Stabilität, insbesondere Lagerstabilität, bevorzugt bei 4 Wochen und 30 °C Lagertemperatur, und/oder die Ästhetik des Produktes in irgendeiner Form, beispielsweise durch Farbveränderung, Ausbildung von feucht-wirkenden Rändern, unscharf werdenden Grenze zwischen den beiden Phasen oder ähnlichem, beeinträchtigt wird.

Überraschenderweise hat sich gezeigt, dass durch eine Formulierung einer Gelphase, bevorzugt formstabilen Gelphase, umfassend mindestens ein Enzym, bevorzugt Enzymgranulat, bevorzugt mindestens ein Amylasegranulat und/oder mindestens ein Proteasegranulat, insbesondere mindestens ein Subtilisin-haltiges Proteasegranulat, welche mit einem körnigen Gemenge einer festförmigen Zusammensetzung, insbesondere einer pulverförmigen Phase kombiniert wird, dieses Ziel erreicht werden kann. Dabei ist es besonders geeignet, wenn das körnige Gemenge einer festförmigen Zusammensetzung, insbesondere die pulverförmige Phase rieselfähig ist, da somit prozessbedingt eine gezieltere Füllung der wasserlöslichen Umhüllung, insbesondere bei Füllung einer durch Tiefziehen hergestellten Kavität, erzielt werden kann. Darüber hinaus kann die optische Erscheinung des körnigen Gemenges einer festförmigen Zusammensetzung, insbesondere des Pulvers im Vergleich zu einer verpressten Tablette besser geändert werden, insbesondere Texturunterschiede, wie grobe und feine Partikel sowie Partikel oder Bereiche mit unterschiedlichen Farben, gesamt oder als farbige Sprenkel, können so zur Verbesserung eines optisch ansprechenden Erscheinungsbildes eingesetzt werden. Das körnige Gemenge der festförmigen Zusammensetzung, insbesondere das Pulver bietet darüber hinaus auch ohne Zusatz von Sprengmitteln eine verbesserte Löslichkeit im Vergleich zu verpressten Tabletten.

Eine Phase im Sinne der vorliegenden Erfindung ist ein räumlicher Bereich, in dem physikalische Parameter und die chemische Zusammensetzung homogen sind. Eine Phase unterscheidet sich von einer anderen Phase durch verschiedene Merkmale, beispielsweise Inhaltsstoffe, physikalische Eigenschaften, äußeres Erscheinungsbild etc. Bevorzugt können verschiedene Phasen optisch unterschieden werden. So ist für den Verbraucher die wenigstens eine feste Phase eindeutig von der wenigstens einen Gelphase zu unterschieden. Weist das erfindungsgemäße Reinigungsmittel mehr als eine feste Phase auf, so können diese ebenfalls jeweils mit dem bloßen Auge voneinander unterschieden werden, weil sie sich beispielsweise in der Farbgebung voneinander unterscheiden. Gleiches gilt, wenn zwei oder mehr Gelphasen vorliegen. Auch in diesem Fall ist eine optische Unterscheidung der Phasen, beispielsweise auf Grund eines Farb- oder Transparenzunterschiedes möglich. Phasen im Sinne der vorliegenden Erfindung sind somit in sich abgeschlossene Bereiche, die vom Verbraucher optisch mit dem bloßen Auge voneinander unterschieden werden können. Die einzelnen Phasen können bei der Verwendung unterschiedliche Eigenschaften aufweisen, wie beispielsweise die Geschwindigkeit, mit der sich die Phase in Wasser löst und somit die Geschwindigkeit und die Reihenfolge der Freisetzung der in der jeweiligen Phase enthaltenen Inhaltsstoffe.

Die wenigstens eine feste Phase der vorliegenden Erfindung umfasst ein körniges Gemenge einer festförmigen Zusammensetzung, insbesondere liegt sie in pulverfömiger und rieselfähiger Form vor. Das erfindungsgemäße Reinigungsmittel umfasst somit wenigstens eine feste pulverförmige und rieselfähige Phase, sowie wenigstens eine Gelphase, umfassend mindestens ein Enzym, bevorzugt Enzymgranulat, bevorzugt mindestens ein Amylasegranulat und/oder mindestens ein Proteasegranulat, insbesondere mindestens ein Subtilisin-haltiges Proteasegranulat, sowie wenigstens ein Polyvinylalkohol, als Gelbildner mindestens PVOH und/oder dessen Derivate, insbesondere bevorzugt mindestens PVOH, sowie wenigstens einen mehrwertigen Alkohol umfasst.

Die Rieselfähigkeit eines körnigen Gemenges, insbesondere eines pulverförmigen Feststoffes, der pulverförmigen Phase, bevorzugt des Pulvers und/oder Granulats betrifft sein Vermögen, unter eigenem Gewicht frei zu rieseln. Die Rieselfähigkeit wird bestimmt, in dem die Auslaufzeit von 1000 ml Reinigungsmittelpulver aus einem genormten, an seiner Auslaufrichtung zunächst verschlossenen Rieseltesttrichter mit einem Auslauf von 16,5 mm Durchmesser durch Messen der Zeit für den vollständigen Auslauf des körnigen Gemenges, insbesondere der pulverförmigen Phase, bevorzugt des Pulvers und/oder Granulats, z.B. des Pulvers nach dem Öffnen des Auslaufs gemessen und mit der Auslaufgeschwindigkeit (in Sekunden) eines Standardprüfsands verglichen wird, dessen Auslaufgeschwindigkeit als 100 % definiert wird. Das definierte Sandgemisch zur Kalibrierung der Rieselapparatur ist trockener Seesand. Dabei wird Seesand mit einem Teilchendurchmesser von 0,4 bis 0,8 mm verwendet, erhältlich beispielsweise von Carl Roth, Deutschland CAS-Nr. [14808-60-7]. Zur Trocknung wird der Seesand vor der Messung 24 h bei 60 °C im Trockenschrank auf einer Platte bei einer maximalen Schichthöhe von 2 cm, getrocknet.

Bevorzugte Ausführungsformen der erfindungsgemäßen festen Phasen weisen einen Schüttwinkel/Böschungswinkel von 26 bis 35, von 27 bis 34, von 28 bis 33, wobei der Schüttwinkel gemäß der unten genannten Methode nach 24 h nach der Herstellung des körnigen Gemenges der festförmigen Zusammensetzung, insbesondere der pulverförmigen festen Phase, bevorzugt des Pulvers und/oder Granulats und Lagerung bei 20 °C bestimmt wird, auf. Solche Schüttwinkel haben den Vorteil, dass die Befüllung der Kavitäten mit der wenigstens einen festen Phase vergleichsweise schnell und präzise erfolgen kann.

Zur Bestimmung des Schüttwinkels (oder auch Böschungswinkel genannt) der wenigstens einen festen Phase wird ein Pulvertrichter mit 400ml Inhalt und einem Ablauf mit einem Durchmesser von 25mm gerade in ein Stativ gehängt. Der Trichter wird mittels eines manuell zu bedienenden Rändelrads mit einer Geschwindigkeit von 80 mm/min nach oben gefahren, so dass das körnige Gemenge, insbesondere die pulverförmige Phase, bevorzugt das Pulver und/oder Granulat, z.B. das Pulver herausrieselt. Dadurch bildet sich ein sogenannter Schüttkegel. Die Schüttkegelhöhe und der Schüttkegeldurchmesser werden für die einzelnen festen Phasen bestimmt. Aus dem Quotienten der Schüttkegelhöhe und dem Schüttkegeldurchmesser * 100 berechnet sich der Böschungswinkel.

Besonders geeignet sind solche körnigen Gemenge einer festförmigen Zusammensetzung, insbesondere solche pulverförmigen Phasen, bevorzugt die Pulver und/oder Granulate, z.B. die Pulver die eine Rieselfähigkeit in % zum vorstehend angegebenen Standardprüfstoff von größer als 40 %, bevorzugt größer als 50, insbesondere größer als 55%, besonders bevorzugt größer als 60%, insbesondere bevorzugt zwischen 63 % und 80 %, beispielsweise zwischen 65 % und 75 % aufweisen. Insbesondere geeignet sind solche körnigen Gemenge einer festförmigen Zusammensetzung, insbesondere solche Pulver und/oder Granulate, die eine Rieselfähigkeit in % zum vorstehend angegebenen Standardprüfstoff von größer als 40 %, bevorzugt größer als 45 %, insbesondere größer als 50 %, besonders bevorzugt größer als 55 %, insbesondere bevorzugt größer als 60 % aufweisen, wobei die Messung der Rieselfähigkeit 24 h nach der Herstellung des Pulvers und Lagerung bei 20 °C durchgeführt wird.

Geringere Werte für die Rieselfähigkeit sind eher nicht geeignet, da aus prozesstechnischer Sicht eine genaue Dosierung des körnigen Gemenges, insbesondere der pulverförmigen Phase, bevorzugt des Pulvers und/oder Granulats, z.B. des Pulvers notwendig ist. Insbesondere die Werte größer 50 %, insbesondere größer 55 %, bevorzugt größer 60 % (wobei die Messung der Rieselfähigkeit 24 h nach der Herstellung des Pulvers und Lagerung bei 20 °C durchgeführt wird) haben sich als vorteilhaft erwiesen, da sich durch die gute Dosierbarkeit der körnigen Gemenge, insbesondere der pulverförmigen Phasen, bevorzugt der Pulver und/oder Granulate, z.B. Pulver nur geringe Schwankungen in der dosierten Menge bzw. der Zusammensetzung ergeben. Die genauere Dosierung führt zu einer gleichbleibenden Produktleistung, wirtschaftliche Verluste durch Überdosierung werden so vermieden. Weiterhin ist es vorteilhaft, dass die körnigen Gemenge, insbesondere die pulverförmige Phase, bevorzugt das Pulver und/oder Granulat, z.B. das Pulver gut dosierbar sind, so erreicht man einen schnelleren Ablauf des Dosierprozesses. Weiterhin wird durch eine solch gute Rieselfähigkeit besser vermieden, dass das körnige Gemenge, insbesondere die pulverförmige Phase, bevorzugt das Pulver und/oder Granulat, z.B. das Pulver auf den Teil der wasserlöslichen Umhüllung gelangt, die für die Herstellung der Siegelnaht vorgesehen ist und daher möglichst körnchenfrei, insbesondere pulverfrei bleiben soll.

Das als feste Phase dienende körnige Gemenge der festförmigen Zusammensetzung der vorliegenden Erfindung liegt bevorzugt in rieselfähiger Form (besonders bevorzugt als rieselfähiges Pulver und/oder rieselfähiges Granulat) vor. Das Mittel der erfindungsgemäßen Portion umfasst somit wenigstens eine feste Phase eines rieselfähigen, körnigen Gemenges einer festförmigen Zusammensetzung, insbesondere eines Pulvers sowie wenigstens eine zuvor definierte Gelphase.

Das erfindungsgemäße Reinigungsmittel umfasst vorzugsweise wenigstens ein Tensid. Dieses Tensid ist ausgewählt aus der Gruppe der anionischen, nichtionischen und kationischen Tenside. Das erfindungsgemäße Reinigungsmittel kann auch Mischungen aus mehreren Tensiden, die aus derselben Gruppe ausgewählt sind, enthalten.

Erfindungsgemäß umfassen die wenigstens eine feste Phase und/oder die wenigstens eine Gelphase wenigstens ein Tensid. Es ist möglich, dass nur die wenigstens eine feste Phase oder nur die wenigstens eine Gelphase wenigstens ein Tensid umfassen. Umfassen beide Phasen ein Tensid, so handelt es sich vorzugsweise um voneinander verschiedene Tenside. Es ist jedoch auch möglich, dass feste und Gelphase dasselbe Tensid oder dieselben Tenside aufweisen. Erfindungsgemäße wenigstens eine feste und/oder Gelphasen enthalten vorzugsweise mindestens ein nichtionisches Tensid. Als nichtionische Tenside können alle dem Fachmann bekannten nichtionischen Tenside eingesetzt werden. Bevorzugt werden schwachschäumende nichtionische Tenside eingesetzt, insbesondere alkoxylierte, vor allem ethoxylierte, schwachschäumende nichtionische Tenside, wie beispielsweise Alkylglykoside, alkoxylierte, vorzugsweise ethoxylierte oder ethoxylierte und propoxylierte Fettsäurealkylester, Polyhydroxyfettsäureamide oder Aminoxide. Besonders bevorzugte nichtionische Tenside werden im Folgenden näher spezifiziert.

Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Mit besonderem Vorzug werden ethoxylierte Niotenside, die aus C₆₋₂₀-Monohydroxyalkanolen oder C₆₋₂₀-Alkylphenolen oder C₁₆₋₂₀-Fettalkoholen und mehr als 12 Mol, vorzugsweise mehr als 15 Mol und insbesondere mehr als 20 Mol Ethylenoxid pro Mol Alkohol gewonnen wurden, eingesetzt. Ein besonders bevorzugtes Niotensid wird aus einem geradkettigen Fettalkohol mit 16 bis 20 Kohlenstoffatomen (C₁₆₋₂₀-Alkohol), vorzugsweise einem C₁₈-Alkohol und mindestens 12 Mol, vorzugsweise mindestens 15 Mol und insbesondere mindestens 20 Mol Ethylenoxid gewonnen. Hierunter sind die sogenannten "narrow range ethoxylates" besonders bevorzugt.

Bevorzugt einzusetzende Tenside stammen aus den Gruppen der alkoxylierten Niotenside, insbesondere der ethoxylierten primären Alkohole und Mischungen dieser Tenside mit strukturell komplizierter aufgebauten Tensiden wie Polyoxypropylen/Polyoxyethylen/Polyoxypropylen ((PO/EO/PO)-Tenside). Solche (PO/EO/PO)-Niotenside zeichnen sich darüber hinaus durch gute Schaumkontrolle aus.

Als besonders bevorzugte Niotenside haben sich im Rahmen der vorliegenden Erfindung für die schwachschäumende Niotenside erwiesen, welche alternierende Ethylenoxid- und Alkylenoxideinheiten aufweisen. Unter diesen sind wiederum Tenside mit EO-AO-EO-AO-Blöcken bevorzugt, wobei jeweils eine bis zehn EO- beziehungsweise AO-Gruppen aneinander gebunden sind, bevor ein Block aus den jeweils anderen Gruppen folgt. Hier sind nichtionisches Tenside der allgemeinen Formel 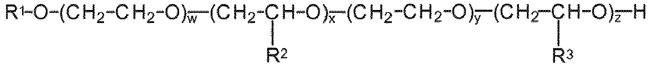 bevorzugt, in der R¹ für einen geradkettigen oder verzweigten, gesättigten oder einbeziehungsweise mehrfach ungesättigten C₆₋₂₄-Alkyl- oder-Alkenylrest steht; jede Gruppe R² beziehungsweise R³ unabhängig voneinander ausgewählt ist aus -CH₃, -CH₂CH₃, -CH₂CH₂-CH₃, -CH(CH₃)₂ und die Indizes w, x, y, z unabhängig voneinander für ganze Zahlen von 1 bis 6 stehen.

Bevorzugte Niotenside der vorstehenden Formel lassen sich durch bekannte Methoden aus den entsprechenden Alkoholen R¹-OH und Ethylen- beziehungsweise Alkylenoxid herstellen. Der Rest R¹ in der vorstehenden Formel kann je nach Herkunft des Alkohols variieren. Werden native Quellen genutzt, weist der Rest R¹ eine gerade Anzahl von Kohlenstoffatomen auf und ist in der Regel unverzweigt, wobei die linearen Reste aus Alkoholen nativen Ursprungs mit 12 bis 18 C-Atomen, zum Beispielaus Kokos-, Palm-, Talgfett- oder Oleylalkohol, bevorzugt sind. Aus synthetischen Quellen zugängliche Alkohole sind beispielsweise die Guerbetalkohole oder in 2-Stellung methylverzweigte beziehungsweise lineare und methylverzweigte Reste im Gemisch, so wie sie üblicherweise in Oxoalkoholresten vorliegen. Unabhängig von der Art des zur Herstellung der in den Mitteln enthaltenen Niotenside eingesetzten Alkohols sind Niotenside bevorzugt, bei denen R¹ in der vorstehenden Formel für einen Alkylrest mit 6 bis 24, vorzugsweise 8 bis 20, besonders bevorzugt 9 bis 15 und insbesondere 9 bis 11 Kohlenstoffatomen steht.

Als Alkylenoxideinheit, die alternierend zur Ethylenoxideinheit in den bevorzugten Niotensiden enthalten ist, kommt neben Propylenoxid insbesondere Butylenoxid in Betracht. Aber auch weitere Alkylenoxide, bei denen R² beziehungsweise R³ unabhängig voneinander ausgewählt sind aus - CH₂CH₂-CH₃ beziehungsweise -CH(CH₃)₂ sind geeignet. Bevorzugt werden Niotenside der vorstehenden Formel eingesetzt, bei denen R² beziehungsweise R³ für einen Rest -CH₃, w und x unabhängig voneinander für Werte von 3 oder 4 und y und z unabhängig voneinander für Werte von 1 oder 2 stehen.

Weitere bevorzugt eingesetzte nichtionische Tenside der festen Phase sind nichtionische Tenside der allgemeinen Formel R¹O(AlkO)ₓM(OAlk)_{y}OR², wobei R¹ und R² unabhängig voneinander für einen verzweigten oder unverzweigten, gesättigten oder ungesättigten, gegebenenfalls hydroxylierten Alkylrest mit 4 bis 22 Kohlenstoffatomen stehen; Alk für einen verzweigten oder unverzweigten Alkylrest mit 2 bis 4 Kohlenstoffatomen steht; x und y unabhängig voneinander für Werte zwischen 1 und 70 stehen; und M für einen Alkylrest aus der Gruppe CH₂, CHR³, CR³R⁴, CH₂CHR³ und CHR³CHR⁴ steht, wobei R³ und R⁴ unabhängig voneinander für einen verzweigten oder unverzweigten, gesättigten oder ungesättigten Alkylrest mit 1 bis 18 Kohlenstoffatomen stehen.

Bevorzugt sind hierbei nichtionische Tenside der allgemeinen Formel

R¹-CH(OH)CH₂-O(CH₂CH₂O)ₓCH₂CHR(OCH₂CH₂)_{y}-CH₂CH(OH)-R²,

wobei R, R¹ und R² unabhängig voneinander für einen Alkylrest oder Alkenylrest mit 6 bis 22 Kohlenstoffatomen; x und y unabhängig voneinander für Werte zwischen 1 und 40 stehen.

Bevorzugt sind hierbei insbesondere Verbindungen der allgemeinen Formel

R¹-CH(OH)CH₂-O(CH₂CH₂O)ₓCH₂CHR(OCH₂CH₂)_{y}O-CH₂CH(OH)-R²,

in denen R für einen linearen, gesättigten Alkylrest mit 8 bis 16 Kohlenstoffatomen, vorzugsweise 10 bis 14 Kohlenstoffatomen steht und n und m unabhängig voneinander Werte von 20 bis 30 aufweisen. Entsprechende Verbindungen können beispielsweise durch Umsetzung von Alkyldiolen HO-CHR-CH₂-OH mit Ethylenoxid erhalten werden, wobei im Anschluss eine Umsetzung mit einem Alkylepoxid zum Verschluss der freien OH-Funktionen unter Ausbildung eines Dihydroxyethers erfolgt.

Bevorzugte nichtionische Tenside sind hierbei solche der allgemeinen Formel R¹-CH(OH)CH₂O-(AO)_{w}-(AO)ₓ-(A"O)_{y}-(A"'O)_{z}-R², in der
- R¹ für einen geradkettigen oder verzweigten, gesättigten oder ein- bzw. mehrfach ungesättigten C₆₋₂₄-Alkyl- oder -Alkenylrest steht;
- R² für Wasserstoff oder einen linearen oder verzweigten Kohlenwasserstoffrest mit 2 bis 26 Kohlenstoffatomen steht;
- A, A', A" und A'" unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) stehen,
- w, x, y und z für Werte zwischen 0,5 und 120 stehen, wobei x, y und/oder z auch 0 sein können.

Durch den Zusatz der vorgenannten nichtionischen Tenside der allgemeinen Formel R¹-CH(OH)CH₂O-(AO)_{w}-(A'O)ₓ-(A"O)_{y}-(A"'O)_{z}-R², nachfolgend auch als "Hydroxymischether" bezeichnet, kann überraschenderweise die Reinigungsleistung erfindungsgemäßer Zubereitungen deutlich verbessert werden und zwar sowohl im Vergleich zu Tensid-freien System wie auch im Vergleich zu Systemen, die alternative nichtionischen Tenside, beispielsweise aus der Gruppe der polyalkoxylierten Fettalkohole enthalten.

Durch den Einsatz dieser nichtionischen Tenside mit einer oder mehreren freien Hydroxylgruppe an einem oder beiden endständigen Alkylresten kann die Stabilität der in den erfindungsgemäßen Reinigungsmittelzubereitungen enthaltenen Enzyme deutlich verbessert werden.

Bevorzugt sind insbesondere solche endgruppenverschlossenen poly(oxyalkylierten) Niotenside, die, gemäß der folgenden Formel 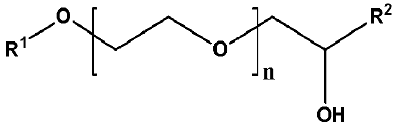 neben einem Rest R¹, welcher für lineare oder verzweigte, gesättigte oder ungesättigte, aliphatische oder aromatische Kohlenwasserstoffreste mit 2 bis 30 Kohlenstoffatomen, vorzugsweise mit 4 bis 22 Kohlenstoffatomen steht, weiterhin einen linearen oder verzweigten, gesättigten oder ungesättigten, aliphatischen oder aromatischen Kohlenwasserstoffrest R² mit 1 bis 30 Kohlenstoffatomen aufweisen, wobei n für Werte zwischen 1 und 90, vorzugsweise für Werte zwischen 10 und 80 und insbesondere für Werte zwischen 20 und 60 steht. Insbesondere bevorzugt sind Tenside der vorstehenden Formel, in denen R¹ für C₇ bis C₁₃, n für eine ganze natürliche Zahl von 16 bis 28 und R² für C₈ bis C₁₂ steht.

Besonders bevorzugt sind Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 sowie y für einen Wert von mindestens 15 steht. Zur Gruppe dieser nichtionischen Tenside zählen beispielsweise die C₂₋₂₆ Fettatkohot-(PO)₁-(EO)₁₅₋₄₀-2-hydroxyalkylether, insbesondere auch die C₈₋₁₀ Fettalkohol-(PO)₁-(EO)₂₂-2-hydroxydecylether.

Besonders bevorzugt sind weiterhin solche endgruppenverschlossenen poly(oxyalkylierten) Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH 3, -CH(CH₃)₂, vorzugsweise jedoch für -CH₃steht, und x und y unabhängig voneinander für Werte zwischen 1 und 32 stehen, wobei Niotenside mit R³= -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel R¹O[CH₂CH(R³)O]ₓ[CH₂]ₖCH(OH)[CH₂]ⱼOR², in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl-, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x > 2 ist, kann jedes R³ in der oben stehenden Formel R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der oben stehenden Formel unterschiedlich sein, falls x > 2 ist. Hierdurch kann die Alkylenoxideinheit in der eckigen Klammer variiert werden. Steht x beispielsweise für 3, kann der Rest R³ ausgewählt werden, um Ethylenoxid- (R³= H) oder Propylenoxid- (R³= CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der oben stehenden Formel weisen Werte von k = 1 und j = 1 auf, so dass sich die vorstehende Formel zu R¹O[CH₂CH(R³)O]ₓCH₂CH(OH)CH₂OR² vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt. Als besonders wirkungsvoll haben sich schließlich die nichtionischen Tenside der allgemeine Formel R¹-CH(OH)CH₂O-(AO)_{w}-R² erwiesen, in der
- R¹ für einen geradkettigen oder verzweigten, gesättigten oder ein- bzw. mehrfach ungesättigten C6-24-Alkyl- oder -Alkenylrest steht;
- R² für einen linearen oder verzweigten Kohlenwasserstoffrest mit 2 bis 26 Kohlenstoffatomen steht;
- A für einen Rest aus der Gruppe CH₂CH₂, CH₂CH₂CH₂, CH₂CH(CH₃), vorzugsweise für CH₂CH₂ steht, und
- w für Werte zwischen 1 und 120, vorzugsweise 10 bis 80, insbesondere 20 bis 40 steht.

Zur Gruppe dieser nichtionischen Tenside zählen beispielsweise die C₄₋₂₂ Fettatkohot-(EO)₁₀₋₈₀-2-hydroxyalkylether, insbesondere auch die C₈₋₁₂ Fettalkohol-(EO)₂₂-2-hydroxydecylether und die C₄₋₂₂ Fettatkohot-(EO)₄₀₋₈₀-2-hydroxyalkylether.

Bevorzugt enthält die wenigstens eine feste und/oder die wenigstens eine Gelphase mindestens ein nichtionisches Tensid, vorzugsweise ein nichtionisches Tensid aus der Gruppe der Hydroxymischether, wobei der Gewichtsanteil des nichtionischen Tensids am Gesamtgewicht der Gelphase vorzugsweise 0,5 Gew.-% bis 30 Gew.-%, bevorzugt 5 Gew.-% bis 25 Gew.-% und insbesondere 10 Gew.-% bis 20 Gew.-% beträgt.

In einer weiteren bevorzugten Ausführungsform ist das nichtionische Tensid der festen und/oder Gelphase ausgewählt aus nichtionischen Tensiden der allgemeinen Formel R¹-O(CH₂CH₂O)ₓCR³R⁴(OCH₂CH₂)_{y}O-R², in der R¹ und R² unabhängig voneinander für einen Alkylrest oder Alkenylrest mit 4 bis 22 Kohlenstoffatomen; R³ und R⁴ unabhängig voneinander für H oder für einen Alkylrest oder Alkenylrest mit 1 bis 18 Kohlenstoffatomen und x und y unabhängig voneinander für Werte zwischen 1 und 40 stehen.

Bevorzugt sind hierbei insbesondere Verbindungen der allgemeinen Formel R¹-O(CH₂CH₂O)ₓCR³R⁴(OCH₂CH₂)_{y}O-R², in der R³ und R⁴ für H stehen und die Indices x und y unabhängig voneinander Werte von 1 bis 40, vorzugsweise von 1 bis 15 annehmen.

Besonders bevorzugt sind insbesondere Verbindungen der allgemeinen Formel R¹-O(CH₂CH₂O)ₓCR³R⁴(OCH₂CH₂)_{y}O-R², in der die Reste R¹ und R² unabhängig voneinander gesättigte Alkylreste mit 4 bis 14 Kohlenstoffatome darstellen und die Indices x und y unabhängig voneinander Werte von 1 bis 15 und insbesondere von 1 bis 12 annehmen.

Weiterhin bevorzugt sind solche Verbindungen der allgemeinen Formel R¹-O(CH₂CH₂O)ₓCR³R⁴(OCH₂CH₂)_{y}O-R², in der einer der Reste R¹ und R² verzweigt ist. Ganz besonders bevorzugt sind Verbindungen der allgemeinen Formel R¹-O(CH₂CH₂O)ₓCR³R⁴(OCH₂CH₂)_{y}O-R², in der die Indices x und y unabhängig voneinander Werte von 8 bis 12 annehmen.

Die angegebenen C-Kettenlängen sowie Ethoxylierungsgrade beziehungsweise Alkoxylierungsgrade der Niotenside stellen statistische Mittelwerte dar, die für ein spezielles Produkt eine ganze oder eine gebrochene Zahl sein können. Aufgrund der Herstellverfahren bestehen Handelsprodukte der genannten Formeln zumeist nicht aus einem individuellen Vertreter, sondern aus Gemischen, wodurch sich sowohl für die C-Kettenlängen als auch für die Ethoxylierungsgrade beziehungsweise Alkoxylierungsgrade Mittelwerte und daraus folgend gebrochene Zahlen ergeben können.

Selbstverständlich können die vorgenannten nichtionischen Tenside (Niotenside) nicht nur als Einzelsubstanzen, sondern auch als Tensidgemische aus zwei, drei, vier oder mehr Tensiden eingesetzt werden.

Insbesondere bevorzugt sind in der wenigstens einen festen Phase solche nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Geeignete nichtionische Tenside, die Schmelz- beziehungsweise Erweichungspunkte im genannten Temperaturbereich aufweisen, sind beispielsweise schwachschäumende nichtionische Tenside, die bei Raumtemperatur fest oder hochviskos sein können. Werden Niotenside eingesetzt, die bei Raumtemperatur hochviskos sind, so ist bevorzugt, dass diese eine Viskosität oberhalb von 20 Pa·s, vorzugsweise oberhalb von 35 Pa·s und insbesondere oberhalb 40 Pa·s aufweisen. Auch Niotenside, die bei Raumtemperatur wachsartige Konsistenz besitzen, sind bevorzugt.

Das bei Raumtemperatur feste Niotensid besitzt vorzugsweise Propylenoxideinheiten (PO) im Molekül. Vorzugsweise machen solche PO-Einheiten bis zu 25 Gew.-%, besonders bevorzugt bis zu 20 Gew.-% und insbesondere bis zu 15 Gew.-% der gesamten Molmasse des nichtionischen Tensids aus. Besonders bevorzugte nichtionische Tenside sind ethoxylierte Monohydroxyalkanole oder Alkylphenole, die zusätzlich Polyoxyethylen-Polyoxypropylen Blockcopolymereinheiten aufweisen. Der Alkohol- beziehungsweise Alkylphenolteil solcher Niotensidmoleküle macht dabei vorzugsweise mehr als 30 Gew.-%, besonders bevorzugt mehr als 50 Gew.-% und insbesondere mehr als 70 Gew.-% der gesamten Molmasse solcher Niotenside aus. Bevorzugte Mittel sind dadurch gekennzeichnet, dass sie ethoxylierte und propoxylierte Niotenside enthalten, bei denen die Propylenoxideinheiten im Molekül bis zu 25 Gew.-%, bevorzugt bis zu 20 Gew.-% und insbesondere bis zu 15 Gew.-% der gesamten Molmasse des nichtionischen Tensids ausmachen. Weitere besonders bevorzugt in der festen Phase einzusetzende Niotenside mit Schmelzpunkten oberhalb Raumtemperatur enthalten 40 bis 70% eines Polyoxypropylen/Polyoxyethylen/Polyoxypropylen-Blockpolymerblends, der 75 Gew.-% eines umgekehrten Block-Copolymers von Polyoxyethylen und Polyoxypropylen mit 17 Mol Ethylenoxid und 44 Mol Propylenoxid und 25 Gew.-% eines Block-Copolymers von Polyoxyethylen und Polyoxypropylen, initiiert mit Trimethylolpropan und enthaltend 24 Mol Ethylenoxid und 99 Mol Propylenoxid pro Mol Trimethylolpropan, enthält.

Der Gewichtsanteil des nichtionischen Tensids am Gesamtgewicht der festen Phase beträgt in einer bevorzugten Ausführungsform von 0,1 bis 20 Gew.-%, besonders bevorzugt von 0,5 bis 15 Gew.-%, insbesondere von 2,5 bis 10 Gew.-%.

Als anionische Tenside eignen sich in den Geschirrspülmitteln alle anionischen oberflächenaktiven Stoffe. Diese sind gekennzeichnet durch eine wasserlöslich machende, anionische Gruppe wie z. B. eine Carboxylat-, Sulfat- oder Sulfonat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Geeignete anionische Tenside liegen vorzugsweise in Form der Natrium-, Kalium- und Ammonium- sowie der Mono-, Di- und Trialkanolammoniumsalze mit 2 bis 4 C-Atomen in der Alkanolgruppe vor, aber auch Zink, Mangan(II), Magnesium, Calcium oder Mischungen hieraus können als Gegenionen dienen. Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül.

An Stelle der genannten Tenside oder in Verbindung mit ihnen können auch kationische und/oder amphotere Tenside, wie Betaine oder quartäre Ammoniumverbindungen, eingesetzt werden. Es ist allerdings bevorzugt, dass keine kationischen und/oder amphoteren Tenside eingesetzt werden.

Tenside beeinflussen die Opazität der Gelphase. In einer anderen Ausführungsform ist die Gelphase daher frei von nichtionischen Tensiden, insbesondere frei von Tensiden. "Frei von" bedeutet, dass die Gelphase weniger als 1,0 Gew.-% und insbesondere weniger als 0,1 Gew.-%, bevorzugt kein Tensid bzw. kein nichtionisches Tensid) enthält.

Bevorzugte erfindungsgemäße Reinigungsmittel sind weiterhin dadurch gekennzeichnet, dass sie in der wenigstens einen festen und/oder der wenigstens einen Gelphase, insbesondere in der festen Phase, weniger als 1,0 Gew.-% und insbesondere weniger als 0,1 Gew.-%, bevorzugt kein anionisches Tensid enthalten.

Gemäß einer besonders bevorzugten Ausführungsform sind die erfindungsgemäßen Reinigungsmittel dadurch gekennzeichnet, dass die wenigstens eine Gelphase weniger als 1 Gew.-%, insbesondere weniger als 0,5 Gew.-%, insbesondere weniger als 0,1 Gew.-% Aniontensid, jeweils bezogen auf das Gesamtgewicht der Gelphase umfasst.

Bevorzugt ist die wenigstens eine Gelphase im Wesentlichen frei von Aniontensiden. Im Wesentlichen frei bedeutet, dass die wenigstens eine Gelphase weniger als 0,05 Gew.-% Aniontensid jeweils bezogen auf das Gesamtgewicht der Gelphase enthält.

Es hat sich in diesem Zusammenhang gezeigt, dass die Anwesenheit von 1 Gew.-% Aniontensid in der wenigstens einen Gelphase zu schlechterem Schaumverhalten sowie schlechterem Klarspülverhalten der Gesamtzusammensetzung führt. Weiterhin beeinflussen höhere Mengen an Aniontensiden die Aushärtung negativ. Gemäß einer besonders bevorzugten Ausführungsform enthält die Gelphase weniger als 1 Gew.-%, bevorzugt weniger als 0,5 Gew.-%, insbesondere weniger als 0,05 Gew.-% Fettsäuresalze bzw. Seifen.

Gemäß einer weiteren Ausführungsform kann die wenigstens eine Gelphase Zucker aufweisen. Zucker umfassen erfindungsgemäß Zuckeralkohole, Monosaccharide, Disaccharide sowie Oligosaccharide. In einer bevorzugten Ausführungsform umfasst die wenigstens eine Gelphase mindestens einen von Glycerin verschiedenen Zuckeralkohol, bevorzugt mindestens ein Monosaccharid- oder Disaccharid-Zuckeralkohol. Insbesondere bevorzugt sind Mannitol, Isomalt, Lactit, Sorbitol, Threit, Erythrit, Arabit und Xylitol. Besonders bevorzugte Monosaccharid-Zuckeralkohole sind Pentitole und/oder Hexitole. Ganz besonders bevorzugt ist Xylitol und/oder Sorbitol.

In einer weiteren Ausführungsform kann die Gelphase Disaccharide, insbesondere Saccharose, umfassen. Der Anteil an Saccharose beträgt 0 Gew.-% bis 30 Gew.-%, insbesondere 5 Gew.-% bis 25 Gew.-%, besonders bevorzugt 10 Gew.-% bis 20 Gew.-%, bezogen auf das Gewicht der Gelphase. In höheren Mengen löst sich der Zucker nicht vollständig in der Gelphase und führt zu einer Trübung derselben. Durch den Einsatz von Zucker, insbesondere in einem Anteil von 10 Gew.-5 bis 15 Gew.-% wird die Feuchteentwicklung reduziert und damit die Haftung zur wenigstens einen festen Phase verbessert.

Der Einsatz von Buildersubstanzen (Gerüststoffen) wie Silikaten, Aluminiumsilikaten (insbesondere Zeolithen), Salze organischer Di- und Polycarbonsäuren sowie Mischungen dieser Stoffe, vorzugsweise wasserlöslicher Buildersubstanzen, kann von Vorteil sein.

In einer erfindungsgemäß besonders bevorzugten Ausführungsform wird auf den Einsatz von Phosphaten (auch Polyphosphaten) weitgehend oder vollständig verzichtet. Das Mittel enthält in dieser Ausführungsform vorzugsweise weniger als 5 Gew.-%, besonders bevorzugt weniger als 3 Gew.-%, insbesondere weniger als 1 Gew.-% Phosphat(e). Besonders bevorzugt ist das Mittel in dieser Ausführungsform völlig phosphatfrei, d.h. die Mittel enthalten weniger als 0,1 Gew.-% Phosphat(e).

Zu den Gerüststoffen zählen insbesondere Carbonate, Citrate, Phosphonate (sofern regulatorisch zulässig), organische Gerüststoffe und Silikate. Der Gewichtsanteil der gesamten Gerüststoffe am Gesamtgewicht erfindungsgemäßer Mittel beträgt vorzugsweise 15 bis 80 Gew.-% und insbesondere 20 bis 70 Gew.-%.

Erfindungsgemäß geeignete organische Gerüststoffe sind beispielsweise die in Form ihrer Natriumsalze einsetzbaren Polycarbonsäuren (Polycarboxylate), wobei unter Polycarbonsäuren solche Carbonsäuren verstanden werden, die mehr als eine, insbesondere zwei bis acht Säurefunktionen, bevorzugt zwei bis sechs, insbesondere zwei, drei, vier oder fünf Säurefunktionen im gesamten Molekül tragen. Bevorzugt sind als Polycarbonsäuren somit Dicarbonsäuren, Tricarbonsäuren Tetracarbonsäuren und Pentacarbonsäuren, insbesondere Di-, Tri- und Tetracarbonsäuren. Dabei können die Polycarbonsäuren noch weitere funktionelle Gruppen, wie beispielsweise Hydroxyl- oder Aminogruppen, tragen. Beispielsweise sind dies Citronensäure, Adipinsäure, Bernsteinsäure, Glutarsäure, Äpfelsäure, Weinsäure, Maleinsäure, Fumarsäure, Zuckersäuren (bevorzugt Aldarsäuren, beispielsweise Galactarsäure und Glucarsäure), Aminocarbonsäuren, insbesondere Aminodicarbonsäuren, Aminotricarbonsäuren, Aminotetracarbonsäuren wie beispielsweise Nitrilotriessigsäure (NTA), Glutamin-N,N-diessigsäure (auch als N,N-Bis(carboxymethyl)-L-glutaminsäure oder GLDA bezeichnet), Methylglycindiessigsäure (MGDA) und deren Derivate sowie Mischungen aus diesen. Bevorzugte Salze sind die Salze der Polycarbonsäuren wie Citronensäure, Adipinsäure, Bernsteinsäure, Glutarsäure, Weinsäure, GLDA, MGDA und Mischungen aus diesen.

Weiterhin geeignet als organische Gerüststoffe sind polymere Polycarboxylate (organische Polymere mit einer Vielzahl, an (insbesondere größer zehn) Carboxylatfunktionen im Makromolekül), Polyaspartate, Polyacetale und Dextrine.

Die freien Säuren besitzen neben ihrer Builderwirkung typischerweise auch die Eigenschaft einer Säuerungskomponente. Insbesondere sind hierbei Citronensäure, Bernsteinsäure, Glutarsäure, Adipinsäure, Gluconsäure und beliebige Mischungen aus diesen zu nennen.

Besonders bevorzugte erfindungsgemäße Reinigungsmittel, insbesondere Geschirrspülmittel, bevorzugt maschinelle Geschirrspülmittel, enthalten als einen ihrer wesentlichen Gerüststoffe ein oder mehrere Salze der Citronensäure, also Citrate. Diese sind vorzugsweise in einem Anteil von 2 bis 40 Gew.-%, insbesondere von 5 bis 30 Gew.-%, besonders von 7 bis 28 Gew.-%, besonders bevorzugt 10 bis 25 Gew.-%, ganz besonders bevorzugt 15 bis 20 Gew.-% enthalten, jeweils bezogen auf das Gesamtgewicht des Mittels.

Besonders bevorzugt ist ebenfalls der Einsatz von Carbonat(en) und/oder Hydrogencarbonat(en), vorzugsweise Alkalicarbonat(en), besonders bevorzugt Natriumcarbonat (Soda), in Mengen von 2 bis 50 Gew.-%, vorzugsweise von 4 bis 40 Gew.-% und insbesondere von 10 bis 30 Gew.-%, ganz besonders bevorzugt 10 bis 24 Gew.-%, jeweils bezogen auf das Gewicht des Mittels.

Besonders bevorzugte erfindungsgemäße Reinigungsmittel, insbesondere Geschirrspülmittel, bevorzugt maschinelle Geschirrspülmittel, sind dadurch gekennzeichnet, dass sie mindestens zwei Gerüststoffe aus der Gruppe der Silikate, Phosphonate (sofern regulatorisch zulässig),, Carbonate, Aminocarbonsäuren und Citrate enthalten, wobei der Gewichtsanteil dieser Gerüststoffe, bezogen auf das Gesamtgewicht des erfindungsgemäßen Reinigungsmittels, bevorzugt 5 bis 70 Gew.-%, vorzugsweise 15 bis 60 Gew.- % und insbesondere 20 bis 50 Gew.-% beträgt. Die Kombination von zwei oder mehr Gerüststoffen aus der oben genannten Gruppe hat sich für die Reinigungs- und Klarspülleistung erfindungsgemäßer Reinigungsmittel, insbesondere Geschirrspülmittel, bevorzugt maschinelle Geschirrspülmittel, als vorteilhaft erwiesen. Über die hier erwähnten Gerüststoffe hinaus können noch ein oder mehrere andere Gerüststoffe zusätzlich enthalten sein.

Bevorzugte Reinigungsmittel, insbesondere Geschirrspülmittel, bevorzugt maschinelle Geschirrspülmittel, sind durch eine Gerüststoffkombination aus Citrat und Carbonat und/oder Hydrogencarbonat gekennzeichnet. In einer erfindungsgemäß ganz besonders bevorzugten Ausführungsform wird eine Mischung aus Carbonat und Citrat eingesetzt, wobei die Menge an Carbonat vorzugsweise von 5 bis 40 Gew.-%, insbesondere 10 bis 35 Gew.-%, ganz besonders bevorzugt 15 bis 30 Gew.-% und die Menge an Citrat vorzugsweise von 5 bis 35 Gew.-%, insbesondere 10 bis 25 Gew.-%, ganz besonders bevorzugt 15 bis 20 Gew.-%, jeweils bezogen auf die Gesamtmenge des Reinigungsmittels, beträgt, wobei die Gesamtmenge dieser beiden Gerüststoffe vorzugsweise 20 bis 65 Gew.-%, insbesondere 25 bis 60 Gew.-%, bevorzugt 30 bis 50 Gew.-%, beträgt. Darüber hinaus können noch ein oder mehrere weitere Gerüststoffe zusätzlich enthalten sein.

Die erfindungsgemäßen Reinigungsmittel, insbesondere Geschirrspülmittel, bevorzugt maschinelle Geschirrspülmittel, können als weiteren Gerüststoff insbesondere Phosphonate (sofern regulatorisch zulässig) enthalten, sofern regulatorisch zulässig. Als Phosphonat-Verbindung wird vorzugsweise ein Hydroxyalkan- und/oder Aminoalkanphosphonat eingesetzt. Unter den Hydroxyalkanphosphonaten ist das 1- Hydroxyethan-1,1-diphosphonat (HEDP) von besonderer Bedeutung. Als Aminoalkanphosphonate kommen vorzugsweise Ethylendiamintetramethylenphosphonat (EDTMP), Diethylentriamin- pentamethylenphosphonat (DTPMP) sowie deren höhere Homologe in Frage. Phosphonate sind in erfindungsgemäßen Mitteln (sofern regulatorisch zulässig) vorzugsweise in Mengen von 0,1 bis 10 Gew.-%, insbesondere in Mengen von 0,5 bis 8 Gew.-%, ganz besonders bevorzugt von 2,5 bis 7,5 Gew.-%, jeweils bezogen auf das Gesamtgewicht des Mittels, enthalten.

Besonders bevorzugt ist der kombinierte Einsatz von Citrat, (Hydrogen-)Carbonat und Phosphonat (sofern regulatorisch zulässig). Diese können in den oben genannten Mengen eingesetzt werden. Insbesondere werden bei dieser Kombination Mengen von, jeweils bezogen auf das Gesamtgewicht des Mittels, 10 bis 25 Gew.-% Citrat, 10 bis 30 Gew.-% Carbonat (oder Hydrogencarbonat), sowie 2,5 bis 7,5 Gew.-% Phosphonat eingesetzt.

Weitere besonders bevorzugte Reinigungsmittel, insbesondere Geschirrspülmittel, bevorzugt maschinelle Geschirrspülmittel, sind dadurch gekennzeichnet, dass sie neben Citrat und (Hydrogen-) Carbonat sowie ggf. Phosphonat mindestens einen weiteren phosphorfreien Gerüststoff enthalten. Insbesondere ist dieser ausgewählt aus den Aminocarbonsäuren, wobei der weitere phosphorfreie Gerüststoff vorzugsweise ausgewählt ist aus Methylglycindiessigsäure (MGDA), Glutaminsäurediacetat (GLDA), Asparaginsäurediacetat (ASDA), Hydroxyethyliminodiacetat (HEIDA), Iminodisuccinat (IDS) und Ethylendiamindisuccinat (EDDS), besonders bevorzugt aus MGDA oder GLDA. Eine besonders bevorzugte Kombination ist beispielsweise Citrat, (Hydrogen-)Carbonat und MGDA sowie ggf. Phosphonat (sofern regulatorisch zulässig).

Der Gew.-%-Anteil des Weiteren phosphorfreien Gerüststoffs, insbesondere des MGDA und/oder GLDA, beträgt vorzugsweise 0 bis 40 Gew.-%, insbesondere 5 bis 30 Gew.-%, vor allem 7 bis 25 Gew.-%. Besonders bevorzugt ist der Einsatz von MGDA bzw. GLDA, insbesondere MGDA, als Granulat. Von Vorteil sind dabei solche MGDA-Granulate, die möglichst wenig Wasser enthalten und/oder eine im Vergleich zum nicht granulierten Pulver geringere Hygroskopizität (Wasseraufnahme bei 25 °C, Normaldruck) aufweisen. Die Kombination von mindestens drei, insbesondere mindestens vier Gerüststoffen aus der oben genannten Gruppe hat sich für die Reinigungs- und Klarspülleistung erfindungsgemäßer Reinigungsmittel, insbesondere Geschirrspülmittel, bevorzugt maschinelle Geschirrspülmittel, als vorteilhaft erwiesen. Daneben können noch weitere Gerüststoffe enthalten sein.

Als organische Gerüststoffe sind weiterhin polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Geeignete Polymere sind insbesondere Polyacrylate, die bevorzugt eine Molekülmasse von 1000 bis 20000 g/mol aufweisen. Aufgrund ihrer überlegenen Löslichkeit können aus dieser Gruppe wiederum die kurzkettigen Polyacrylate, die Molmassen von 1100 bis 10000 g/mol, und besonders bevorzugt von 1200 bis 5000 g/mol, aufweisen, bevorzugt sein.

Der Gehalt der erfindungsgemäßen Reinigungsmittel, insbesondere Geschirrspülmittel, bevorzugt maschinelle Geschirrspülmittel, an (homo)polymeren Polycarboxylaten beträgt vorzugsweise 0,5 bis 20 Gew.-%, bevorzugt 2 bis 15 Gew.-% und insbesondere 4 bis 10 Gew.-%.

Erfindungsgemäße Reinigungsmittel, insbesondere Geschirrspülmittel, bevorzugt maschinelle Geschirrspülmittel, können als Gerüststoff weiterhin kristalline schichtförmige Silikate der allgemeinen Formel NaMSiₓO₂ₓ₊₁·yH₂O, worin M Natrium oder Wasserstoff darstellt, x eine Zahl von 1,9 bis 22, vorzugsweise von 1,9 bis 4, wobei besonders bevorzugte Werte für x 2, 3 oder 4 sind, und y für eine Zahl von 0 bis 33, vorzugsweise von 0 bis 20 steht. Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche vorzugsweise löseverzögert sind und Sekundärwascheigenschaften aufweisen.

In Ergänzung zu den vorgenannten Gerüststoffen können die erfindungsgemäßen Reinigungsmittel weiterhin Alkalimetallhydroxide enthalten. Diese Alkaliträger werden in den Reinigungsmitteln und insbesondere in den wenigstens einen Gelphasen bevorzugt nur in geringen Mengen, vorzugsweise in Mengen unterhalb 10 Gew.-%, bevorzugt unterhalb 6 Gew.-%, vorzugsweise unterhalb 5 Gew.-%, besonders bevorzugt zwischen 0,1 und 5 Gew.-% und insbesondere zwischen 0,5 und 5 Gew.-%, jeweils bezogen auf das Gesamtgewicht des Reinigungsmittels eingesetzt. Alternative erfindungsgemäße Reinigungsmittel sind frei von Alkalimetallhydroxiden.

Neben den bisher angeführten Bestandteilen können die wenigstens eine feste und/oder die wenigstens eine Gelphase des erfindungsgemäßen Reinigungsmittels weitere Inhaltsstoffe enthalten. Hierzu zählen beispielsweise anionische, kationische und/oder amphotere Tenside, Bleichmittel, Bleichaktivatoren, Bleichkatalysatoren, weitere Lösungsmittel, Verdicker, Sequestrierungsmittel, Elektrolyte, Korrosionsinhibitoren, insbesondere Silberschutzmittel, Glaskorrosionsinhibitoren, Schauminhibitoren, Farbstoffe, Duftstoffe (insbesondere in der wenigstens einen festen Phase), Additive zur Verbesserung des Ablauf- und Trocknungsverhaltens, zur Einstellung der Viskosität, zur Stabilisierung, UV-Stabilisatoren, Konservierungsmittel, antimikrobielle Wirkstoffe (Desinfektionsmittel), pH-Stellmittel in Mengen von üblicherweise nicht mehr als 5 Gew.-% enthalten sein.

Als weiteres Lösungsmittel enthalten erfindungsgemäße Mittel vorzugsweise mindestens ein Alkanolamin. Das Alkanolamin ist hierbei vorzugsweise ausgewählt aus der Gruppe bestehend aus Mono-, Di-, Triethanol- und -Propanolamin und deren Mischungen. Das Alkanolamin ist in erfindungsgemäßen Mitteln vorzugsweise in einer Menge von 0,5 bis 10 Gew.-%, insbesondere in einer Menge von 1 bis 6 Gew.-%, enthalten. In bevorzugten Reinigungsmitteln ist die wenigstens eine Gelphase im wesentlichen frei von Alkanolamin, d.h. die wenigstens eine Gelphase enthält weniger als 1 Gew.-%, insbesondere weniger als 0,5 Gew.-%, bevorzugt weniger als 0,1 Gew.-%, insbesondere bevorzugt weniger als 0,05 Gew.-% Alkanolamin und das Alkanolamin ist nur in der wenigstens einen festen Phase enthalten.

Gemäß einer weiteren bevorzugten Ausführungsform umfasst die mindestens eine gelförmige Phase des Reinigungsmittel, insbesondere Geschirrspülmittels, bevorzugt maschinellen Geschirrspülmittels, mindestens ein wasserlösliches Zinksalz, bevorzugt Zinkchlorid, Zinksulfat und/oder Zinkacetat, besonders bevorzugt Zinkacetat, beispielsweise Zinkacetat-Anhydrat, bevorzugt in einer Menge von 0,05 bis 3 Gew.-%, insbesondere von 0,1 bis 2,4 Gew.-%, ganz besonders bevorzugt von 0,2 bis 1,0 Gew.-%, bezogen auf das Gesamtgewicht der gelförmigen Phase, enthält.

Für eine gute Einarbeitungsfähigkeit der Zinksalze, insbesondere von Zinksulfat und/oder Zinkacetat, insbesondere von Zinkacetat (z.B. in der wasserfreien Form des Salzes) in wasserarme Gelphasen, welche carboxylat- und/oder sulfonsäuregruppenhaltige Polymere aufweisen, ist es besonders bevorzugt, wenn die Menge an Zinksalz in der wasserfreien Gelphase von 0,2 bis 1,0 Gew.-%, beispielsweise 0,5 Gew.-% gewählt wird.

Zusätzlich zu den o.g. Zinksalzen können Polyethylenimine oder Polyvinylamine, wie sie beispielsweise unter dem Namen Lupasol® (BASF) erhältlich sind, vorzugsweise in einer Menge von 0 bis 5 Gew.-%, insbesondere 0,01 bis 2 Gew.-%, als Glaskorrosionsinhibitoren eingesetzt werden.

Als Additive geeignete Polymere sind insbesondere Maleinsäure-Acrylsäure-Copolymer-Na-Salz (beispielsweise Sokalan® CP 5 der Firma BASF, Ludwigshafen (Deutschland)), modifiziertes Polyacrylsäure-Na-Salz (beispielsweise Sokalan® CP 10 der Firma BASF, Ludwigshafen (Deutschland)), modifiziertes Polycarboxylat-Na-Salz (beispielsweise Sokalan® HP 25 der Firma BASF, Ludwigshafen (Deutschland)), Polyalkylenoxid, modifiziertes Heptamethyltrisiloxan (beispielsweise Silwet® L-77 der Firma BASF, Ludwigshafen (Deutschland)), Polyalkylenoxid, modifiziertes Heptamethyltrisiloxan (beispielsweise Silwet® L-7608 der Firma BASF, Ludwigshafen (Deutschland)) sowie Polyethersiloxane (Copolymere von Polymethylsiloxanen mit Ethylenoxid-/Propylenoxidsegmenten (Polyetherblöcken)), vorzugsweise wasserlösliche lineare Polyethersiloxane mit terminalen Polyetherblöcken wie Tegopren® 5840, Tegopren® 5843, Tegopren® 5847, Tegopren® 5851, Tegopren® 5863 oder Tegopren® 5878 der Firma Evonik, Essen (Deutschland). Als Additive geeignete Buildersubstanzen sind insbesondere Polyasparaginsäure-Na-Salz, Ethylendiamintriacetatkokosalkylacetamid (beispielsweise Rewopol® CHT 12 der Firma Evonik, Essen (Deutschland)), Methylglycindiessigsäure-Tri-Na-Salz und Acetophosphonsäure. Mischungen mit tensidischen oder polymeren Additiven zeigen im Falle von Tegopren® 5843 und Tegopren® 5863 Synergismen. Der Einsatz der Tegopren-Typen 5843 und 5863 ist jedoch bei der Anwendung auf harte Oberflächen aus Glas, insbesondere Glasgeschirr, weniger bevorzugt, da diese Silikontenside auf Glas aufziehen können. In einer besonderen Ausführungsform der Erfindung wird auf die genannten Additive verzichtet.

Ein bevorzugtes Reinigungsmittel, insbesondere maschinelles Geschirrspülmittel, umfasst vorzugsweise weiterhin ein Bleichmittel, insbesondere ein Sauerstoffbleichmittel sowie gegebenenfalls einen Bleichaktivator und/oder Bleichkatalysator. Diese sind, soweit vorhanden, ausschließlich in der wenigstens einen festen Phase enthalten.

Als bevorzugtes Bleichmittel enthalten erfindungsgemäße Reinigungsmittel ein Sauerstoffbleichmittel aus der Gruppe Natriumpercarbonat, Natriumperborattetrahydrat und Natriumperboratmonohydrat. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Weiterhin können auch Bleichmittel aus der Gruppe der organischen Bleichmittel eingesetzt werden. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Wegen seiner guten Bleichleistung wird das Natriumpercarbonat besonders bevorzugt. Ein besonders bevorzugtes Sauerstoffbleichmittel ist Natriumpercarbonat.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die 0- und/oder N-Acylgruppen der genannten C-Atomzahl und/oder gegebenenfalls substituierte Benzoylgruppen tragen. Bevorzugt werden mehrfach acylierte Alkylendiamine, wobei sich Tetraacetylethylendiamin (TAED) als besonders geeignet erwiesen hat.

Bei den Bleichkatalysatoren handelt es sich um bleichverstärkende Übergangsmetallsalze beziehungsweise Übergangsmetallkomplexe wie beispielsweise Mn-, Fe-, Co-, Ru - oder Mo-Salenkomplexe oder - carbonylkomplexe. Auch Mn-, Fe-, Co-, Ru-, Mo-, Ti-, V- und Cu-Komplexe mit N-haltigen Tripod-Liganden sowie Co-, Fe- Cu- und Ru-Amminkomplexe sind als Bleichkatalysatoren verwendbar. Mit besonderem Vorzug werden Komplexe des Mangans in der Oxidationsstufe II, III, IV oder IV eingesetzt, die vorzugsweise einen oder mehrere makrocyclische(n) Ligand(en) mit den Donorfunktionen N, NR, PR, O und/oder S enthalten. Vorzugsweise werden Liganden eingesetzt, die Stickstoff-Donorfunktionen aufweisen. Dabei ist es besonders bevorzugt, Bleichkatalysator(en) in den erfindungsgemäßen Mitteln einzusetzen, welche als makromolekularen Liganden 1,4,7-Trimethyl-1,4,7-triazacyclononan (Me-TACN), 1,4,7-Triazacyclononan (TACN), 1,5,9-Trimethyl-1,5,9-triazacyclododecan (Me-TACD), 2-Methyl-1-1,4,7-trimethyl-1,4,7-triazacyclononan (Me/Me-TACN) und/oder 2-Methyl-1,4,7-triazacyclononan (Me/TACN) enthalten. Geeignete Mangankomplexe sind beispielsweise [Mn^{III}₂(µ-O)₁(µ-OAc)₂(TACN)₂](ClO₄)₂, [Mn^{III}Mn^{IV}(µ-O)₂(µ-OAc)₁(TACN)₂](BPh₄)2, [Mn^{III}₄(µ-O)₆(TACN)₄](ClO₄)₄, [Mn^{III}₂(µ-O)₁(µ-OAc)₂(Me-TACN)₂](ClO₄)₂, [Mn^{III}Mn^{IV}(µ-O)₁(µ-OAc)₂(Me-TACN)₂](ClO₄)₃, [Mn^{IV}2(µ-O)₃(Me-TACN)₂](PF₆)₂ und [Mn^{IV}₂(_{µ}-O)₃(Me/Me-TACN)₂](PF₆)₂(mit OAc = OC(O)CH₃).

Das erfindungsgemäße Reinigungsmittel umfasst bevorzugt wenigstens eine feste Phase und wenigstens eine Gelphase. Das Reinigungsmittel kann somit eine, zwei, drei oder mehr voneinander verschiedene feste Phasen aufweisen; ebenso kann es eine, zwei, drei oder mehr voneinander verschiedene Gelphasen aufweisen. Bevorzugt umfasst das erfindungsgemäße Reinigungsmittel eine feste Phase und eine Gelphase. Besonders bevorzugt umfasst das Reinigungsmittel zwei feste Phasen und eine Gelphase. Bevorzugt umfasst es zwei feste Phasen und zwei Gelphasen. Weiterhin bevorzugt ist eine Ausführungsform, in der das Reinigungsmittel drei feste Phasen und eine oder zwei Gelphasen umfasst.

Dabei beträgt das Gewichtsverhältnis der Gesamtheit der wenigstens einen festen Phase zur Gesamtheit der wenigstens einen Gelphase in der Regel 40:1 bis 2:1, insbesondere 20:1 bis 4:1, bevorzugt 14:1 bis 6:1 beispielsweise 12:1 bis 8:1. Das Gesamtgewicht aller Phase in einer Reinigungsmittelportion kann zwischen 8 und 30 g, insbesondere 10 bis 25 g, bevorzugt 12 bis 21 g, beispielsweise 13 bis 17 g pro Reinigungsmittelportion betragen. In diesem Gewichtsverhältnis ergibt sich eine gute Konzentration an den jeweiligen Inhaltsstoffen der festen beziehungsweise der Gelphase in einem Reinigungsvorgang.

Gemäß einer bevorzugten Ausführungsform ist die Protease in einer Gesamtmenge von 0,001 bis 1000 mg, weiter bevorzugt 0,1 bis 600 mg und noch weiter bevorzugt 1,0 bis 400 mg Enzymprotein (bezogen auf aktives Enzymprotein) in der Reinigungsmittelportion enthalten ist.

Erfindungsgemäß grenzen die wenigstens eine feste Phase und die wenigstens eine Gelphase voll- oder teilflächig aneinander. Dabei ist es bevorzugt, dass die beiden Phasen unmittelbar aneinandergrenzen. Grenzen die wenigstens eine feste Phase und die wenigstens eine Gelphase unmittelbar voll- oder teilflächig aneinander, ist die Stabilität neben einer möglichst kurzen Erstarrungszeit der wenigstens einen Gelphase wichtig. Stabilität bedeutet hier, dass in der Gelphase enthaltende Bestandteile nicht in die wenigstens eine feste Phase übertreten, sondern auch nach einer längeren Lagerung die wenigstens eine feste Phase und die Gelphase optisch getrennt voneinander vorliegen und nicht miteinander in Wechselwirkung treten, wie z.B. Diffusion flüssiger Bestandteile von einer in die andere Phase oder Reaktion von Bestandteilen einer Phase mit denen in der anderen Phase

Ein weiterer Gegenstand der vorliegenden Anmeldung ist ein Verfahren zur Reinigung von harten Oberflächen, insbesondere von Geschirr, in dem die Oberfläche in an sich bekannter Weise unter Verwendung eines erfindungsgemäßen Reinigungsmittels bearbeitet wird. Insbesondere wird dabei die Oberfläche mit dem erfindungsgemäßen Reinigungsmittel in Kontakt gebracht. Die Reinigung erfolgt dabei insbesondere mit einer Reinigungsmaschine, bevorzugt mit einer Geschirrspülmaschine.

Ein weiterer Gegenstand der vorliegenden Erfindung ist ebenso die Verwendung eines Reinigungsmittels, wie vorstehend beschrieben, zur Reinigung von harten Oberflächen, insbesondere von Geschirr, insbesondere in maschinellen Geschirrspülmaschinen.

Für die Verwendung bzw. das Verfahren gilt entsprechend das vorstehend für die Reinigungsmittel konkret offenbarte.

Eine besonders bevorzugte Ausführungsform der Erfindung betrifft ein Reinigungsmittel, welches mindestens ein Enzym, bevorzugt ein Enzymgranulat, Polyethylenglykole mit einer mittleren Molmasse von 200 bis 600 g/mol, Glyzerin, PVOH und wenigstens ein C₃- bis C₅-Alkandiol umfasst. Bevorzugt ist eine Reinigungsmittelmalportion in einer wasserlöslichen Umhüllung, welches mindestens ein Enzym, bevorzugt ein Enzymgranulat, Polyethylenglykole mit einer mittleren Molmasse von 200 bis 600 g/mol, Glyzerin, PVOH und wenigstens ein C₃- bis C₅-Alkandiol umfasst.

Soweit in der vorliegenden Anmeldung festgehalten ist, dass das erfindungsgemäße Reinigungsmittel im Ganzen oder in der wenigstens einen festen Phase oder in der wenigstens einen Gelphase etwas umfasst, ist ebenfalls als offenbart anzusehen, dass Reinigungsmittel bzw. die jeweilige Phase daraus bestehen kann. Im nachfolgenden Ausführungsbeispiel wird das erfindungsgemäße Reinigungsmittel in nicht limitierender Weise beschrieben.

### Ausführungsbeispiele:

Es wurden erfindungsgemäße Reinigungsmittel hergestellt, die eine feste Phase und eine Gelphase umfassten. Hierbei wurden unterschiedliche Geometrien realisiert. Weiterhin wurden Reinigungsmittel hergestellt, die zwei feste Phasen und eine Gelphase umfassten. Ebenfalls wurden Reinigungsmittel hergestellt, die eine feste Phase sowie 3, 4 und 5 Gelphasen, (gleicher oder unterschiedlicher Zusammensetzung) umfassten. Die folgenden Angaben beziehen sich auf Gew.-% Aktivsubstanz bezogen auf das Gesamtgewicht der jeweiligen Phase (sofern nichts anderes angegeben).

**Tabelle 1: Die festen körnigen Gemenge einer festförmigen Zusammensetzung, insbesondere pulverförmigen und rieselfähigen Phasen wiesen dabei die folgende bevorzugte Zusammensetzung auf:**

| | Gew.-% |
|---|---|
| Citrat, Na-Salz | 15-20 |
| Phosphonat (z.B. HEDP) | 0-7,5 |
| Sofern regulatorisch zulässig | (2,5-7,5) |
| MGDA, Na-Salz | 0-25 |
| Disilicat, Na-Salz | 5-35 |
| Soda | 10-25 |
| SILBERSCHUTZ (z.B. Cystein) | 0,0 - 1,0 |
| Percarbonat, Na-Salz | 10-15 |
| Bleichkatalysator (bevorzugt Mn-basiert) | 0,02-0,5 |
| Bleichaktivator (z.B. TAED) | 1-3 |
| Nichtionische(s) Tensid(e), z.B. Fettalkoholalkoxylat, bevorzugt 20-40 EO, ggf. endcapped | 2,5-10 |
| Polycarboxylat | 4-10 |
| Kationisches Copolymer | 0 - 0,75 |
| Disintegrant - (z.B. Crosslinked PVP) | 0-1,5 |
| Protease-Zubereitung (tq) | 0-5 |
| Amylase-Zubereitung (tq) | 0-3 |
| Parfüm | 0,05-0,25 |
| Farbstofflösung | 0,0-1 |
| Zn-Salz (z.B. Acetat) | 0,1-0,3 |
| Natriumsulfat | 0,0 - 10 |
| Wasser | 0,0-1,5 |
| pH-Stellmittel (z.B. Citronensäure) | 0-1,5 |
| Prozesshilfsmittel | 0-5 |

**Tabelle 2: Die dabei eingesetzten Gelphasen hatten die folgenden Zusammensetzungen (Angaben jeweils bezogen auf die Gesamtmenge der Gelphase):**

| | Gew.-% |
|---|---|
| Enzymgranulat (Protease), bezogen auf Aktivproteingehalt | 0,01-7,5 |
| Enzymgranulat (Amylase), bezogen auf Aktivproteingehalt | 0-5,0 |
| Wasserlösliches Zink-Salz (bevorzugt Zinkacetat-Anhydrat) | 0,1-2,4 |
| Glyzerin | 10-50 |
| Propandiol (bevorzugt 1,3-Propandiol) | 10-50 |
| Polycarboxylat Copolymer mit Sulfonsäurehaltigen Gruppen, bevorzugt Acusol® 588 bzw. Acusol ® 590 | 0-30 |
| Nichtionische(s) Tensid(e), z.B. Fettalkoholalkoxylat, bevorzugt 20-40 EO, ggf. endcapped | 0-40 |
| Polyethylenglykol mittl. Mr 200-600, (beispielsweise PEG 400 (INCI) | 8-26 |
| PVOH | 8-22 |
| Prozesshilfsmittel | 0-10 |
| Farbstofflösung | 0,0-1,5 |
| Misc., weitere Aktivstoffe, organische Lösungsmittel, Parfüm, | Add 100 |

Die festen und die Gelphasen konnten beliebig miteinander kombiniert werden. Die räumliche Ausgestaltung der Gelphase, die nach dem Vermischen der Inhaltsstoffe flüssig und innerhalb einer Erstarrungszeit von maximal 10 Minuten formstabil war, wurde durch die räumliche Ausgestaltung der festen Phase sowie durch handelsübliche oder selbst designte Formen vorgegeben. Es wurde eine wasserlösliche Umhüllung in Form eines offenen Pouches durch Tiefziehen einer PVOH-haltigen Folie hergestellt. In diese offene Kavität wurde eine flüssige Zusammensetzung gegossen, welche nach dem Aushärten die Gelphase ergab, darauf wurden festen Phasen in Form eines rieselfähigen Feststoffes in einem Pouch umfassend Polyvinylalkohol eingefüllt und der offene Pouch dann durch Auflegen einer zweiten Folie und Versiegelung mittels Heißsiegelung versiegelt.

**Tabelle 3: Zusammensetzungen der Gelphase in Gew.-%**

| | G1 | G2 | G3 | G4 | G5 | G6 |
|---|---|---|---|---|---|---|
| Zinkacetat wasserfrei | 0,5 | 0,5 | 1,0 | 1,0 | 2,0 | 2,0 |
| Polymer umfassend Acrylsäure- und Amidopropylsulfonsäure-haltige Monomere, Acusol® 588 | 11 | 0 | 0 | 11 | 11 | 0 |
| Polymer umfassend Acrylsäure- und Amidopropylsulfonsäure-haltige Monomere, Acusol® 590 | 0 | 11 | 11 | 0 | 0 | 11 |
| Glycerin | 25 | 25 | 25 | 25 | 25 | 25 |
| 1,3 Propandiol | 30 | 30 | 30 | 30 | 30 | 30 |
| PEG 400 | 15 | 15 | 15 | 15 | 15 | 15 |
| PVOH (Mowiol 4-88) | 15 | 15 | 15 | 15 | 15 | 15 |
| Misc (u.a Prozesshilfsmittel, pH-Stellmittel, Parfum, Farbstoff) | Add 100 | Add 100 | Add 100 | Add 100 | Add 100 | Add 100 |

Gemäß Tabelle 3 wurden entsprechende Formulierungen hergestellt. Die Gelphasen wurden bei Temperaturen von 110 °C gerührt. In die noch heißen Zusammensetzungen wurden kurz vor der Dosierung der Gelphasen jeweils Proteasegranulat 1 oder 2 zudosiert.

Die entsprechenden Gelphasen (1,5 g) wurden anschließend wie unter Tabelle 2 beschrieben zusammen mit einer festen Phase gemäß Tabelle 4 (17,0 g) in Einmalportionen (Pouch) mit einem Gesamtgewicht von 18,5 g konfektioniert.

**Tabelle 4: Zusammensetzung feste Phase:**

| | Gew.-% Aktivsubstanz |
|---|---|
| Citrat, Na-Salz | 35 |
| Soda | 25 |
| Percarbonat, Na-Salz | 10 |
| Bleichkatalysator (Mn-TACN) | 0,05 |
| Bleichaktivator (TAED) | 1 |
| Nichtionische Tenside | 2,5 |
| Polycarboxylat, (Acusol® 588) | 5 |
| Amylase-Zubereitung (tq) | 1,5 |
| Natriumsulfat, Farbstoff, Parfüm | Add 100 |

### Reinigungsleistung:

Mit den so hergestellten Einmalportionen (Gelphase G1, feste Phase, Tabelle 4) wurde die Reinigungsleistung der Zusammensetzungen nach der IKW-Methode in einer Miele GSL, Programm Eco 45°, bei 21 °dH, bestimmt.

**Tabelle 5:**

| | Reinigungsleistung | Creme Brûlée | Eigelb | Spaghetti |
|---|---|---|---|---|
| E1 | G1+Proteasegranulat 1 (30 mg/job) | 7,2 | 4,7 | 3,5 |
| E2 | G1+Proteasegranulat 2 (30 mg/job) | 8,4 | 8,0 | 4,5 |

Beide Proteasegranulate enthalten jeweils 30 mg Aktivprotein / Job (entsprechend Gehalt an aktivem Enzymprotein pro Einmalportion geeignet für eine Reinigungsanwendung). Das Proteasegranulat 1 enthält eine Kombination aus Protease 1a (Protease gemäß SEQ ID NO:6 aus WO2016000973) und Protease 1b (Ovozyme®, ex Novozymes) im Verhältnis 1,5:1. Das Proteasegranulat 2 enthält eine Protease gemäß SEQ ID NO:5 aus WO2017215925.

Überraschenderweise wurde gefunden, dass die Proteasen die noch verbleibenden hohen Temperaturen des Gelansatzes vor dessen Verfestigung (> 90 °C)

Weiterhin wurde überraschend festgestellt, dass bei Einsatz von Proteasegranulat 2 neben einer besonders guten Reinigungsleistung auf proteinhaltige Anschmutzungen auch ein Effekt auf die stärkebasierte Anschmutzung Spaghetti zu beobachten ist.

In einem entsprechenden weiteren Versuch mit den gleichen Parametern wurden lediglich eine geringere Proteasemengen in der Gelphase eingesetzt (vgl. Tabelle 6):

**Tabelle 6:**

| | Reinigungsleistung | Eingebranntes Hackfleisch | Eigelb |
|---|---|---|---|
| E3 | G1+Proteasegranulat 1 (15 mg/job) | 4,2 | 3,2 |
| E4 | G1+Proteasegranulat 2 (15 mg/job) | 6,9 | 5,6 |

Beide Proteasegranulate enthalten jeweils 15 mg Aktivprotein / Job (entsprechend Gehalt an aktivem Enzymprotein pro Einmalportion geeignet für eine Reinigungsanwendung). Das Proteasegranulat 1 enthält eine Kombination aus Protease 1a (Protease gemäß SEQ ID NO:6 aus WO2016000973) und Protease 1b (Ovozyme®, ex Novozymes) im Verhältnis 1:1. Das Proteasegranulat 2 enthält eine Protease gemäß SEQ ID NO:5 aus WO2017215925.

Auch hier wurde überraschend festgestellt, dass beide Proteasen gute Ergebnisse auf proteasesensitiven Anschmutzungen lieferten, insbesondere bei Einsatz von Proteasegranulat 2 ist eine besonders gute Reinigungsleistung insbesondere auf Eigelb und eingebranntes Hackfleisch zu beobachten.

## Patentansprüche

1. Reinigungsmittel, bevorzugt Geschirrspülmittel, insbesondere maschinelles Geschirrspülmittel, bevorzugt phosphatfreies maschinelles Geschirrspülmittel, umfassend mindestens eine gelförmige Phase, insbesondere wasserarme, bevorzugt im wesentlichen wasserfreie gelförmige Phase, welche mindestens ein Enzym, bevorzugt als Granulat, enthält.

2. Reinigungsmittel nach Anspruch 1, wobei der Gewichtsanteil aller Enzyme, bezogen auf die Menge an aktivem Enzymprotein, in der mindestens einen gelförmigen Phase, bezogen auf das Gesamtgewicht der gelförmigen Phase(n), 0,0005 bis 20,0 Gew.-%, bevorzugt 0,001 bis 15, Gew.-%, weiter bevorzugt 0,01 bis 10,0 Gew.-% und noch weiter bevorzugt 0,1 bis 7,5 Gew.-% beträgt.

3. Reinigungsmittel nach Anspruch 1 oder 2, **dadurch gekennzeichnet, dass** das mindestens eine Enzym ausgewählt ist aus der Gruppe der Proteasen, insbesondere aus der Gruppe der Serinproteasen, besonders bevorzugt mindestens eine Protease vom Subtilisin-Typ, und/oder Amylasen, bevorzugt alpha-Amylasen.

4. Reinigungsmittel nach Anspruch 1 bis 3, **dadurch gekennzeichnet, dass** das mindestens eine Enzym ausgewählt ist aus der Gruppe der Proteasen, wobei mindestens eine Protease
a) eine Aminosäuresequenz umfasst, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt eine der folgenden Aminosäuresubstitutionsvarianten, jeweils bezogen auf die Nummerierung gemäß SEQ ID NO:1, aufweist:
(ix) I43V;
(x) M122L, N154S und T156A;
(xi) M211N und P212D;
(xii) M211L und P212D;
(xiii) G160S;
(xiv) D127P, M211L und P212D;
(xv) P212H; oder
(xvi) Q12L, M122L und A222S;
b) eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80%, bevorzugt zu mindestens 90 %, insbesondere zu 100 % identisch ist und mindestens eine Aminosäuresubstitution an einer der Positionen 9, 15, 66, 212 und 239 in der Zählung gemäß SEQ ID NO:2 aufweist, vorzugsweise ausgewählt aus der Gruppe bestehend aus S9R, A15T, V66A, N212D und Q239R;
c) eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80% bevorzugt zu mindestens 90 %, insbesondere zu 100 % identisch ist und eine Aminosäuresubstitution an der Position 97 und eine Insertion von einer Aminosäure zwischen den Aminosäuren an Positionen 97 und 98 in der Zählung gemäß SEQ ID NO:2 aufweist, vorzugsweise ausgewählt aus S97A und/oder S97AD;
d) eine Aminosäuresequenz umfasst, die eine zu der in SEQ ID NO:3 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer der folgenden Positionen 32, 33, 48-54, 58-62, 94-107, 116, 123-133, 150, 152-156, 158-161, 164, 169, 175-186, 197, 198, 203-216 in der Zählung gemäß SEQ ID NO:3 aufweist, insbesondere mindestens eine Aminosäuresubstitution an einer, zwei, drei oder vier der folgenden Positionen 116, 126, 127, 128 und 160 in der Zählung gemäß SEQ ID NO:3, vorzugsweise mindestens eine, noch bevorzugter mehrere, am bevorzugtesten jede der Aminosäuresubstitution G116V, S126L, P127Q und/oder S128A und/oder
e) eine Aminosäuresequenz umfasst, die eine alkalische Protease aus Bacillus lentus DSM 5483 umfasst, insbesondere eine, die eine Aminosäuresequenz aufweist, die zu der in SEQ ID NO:4 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer, zwei, drei oder vier der folgenden Positionen 3, 4, 99 und 199 in der Zählung gemäß SEQ ID NO:4 aufweist, insbesondere die Aminosäuresubstitution R99E oder R99D, sowie optional zusätzlich mindestens eine oder zwei, vorzugsweise alle drei der Aminosäuresubstitutionen S3T, V4I und V199I;

5. Reinigungsmittel nach Anspruch 3 oder 4, **dadurch gekennzeichnet, dass** der Gewichtsanteil der mindestens einen Protease, bezogen auf die Menge an aktivem Enzymprotein aller Proteasen, am Gesamtgewicht des Reinigungsmittels 0,005 bis 5 Gew.-%, bevorzugt 0,01 bis 2,0 Gew.-%, weiter bevorzugt 0,05 bis 1,5 Gew.-% und noch weiter bevorzugt 0,01 bis 1,0 Gew.-% beträgt.

6. Reinigungsmittel nach Anspruch 3 bis 5, **dadurch gekennzeichnet, dass** der Gewichtsanteil aller Proteasen, bezogen auf die Menge an aktivem Enzymprotein, in der mindestens einen gelförmigen Phase, bezogen auf das Gesamtgewicht der gelförmigen Phase(n), 0,0005 bis 20,0 Gew.-%, bevorzugt 0,001 bis 15, Gew.-%, weiter bevorzugt 0,01 bis 10,0 Gew.-% und noch weiter bevorzugt 0,1 bis 7,5 Gew.-% beträgt.

7. Reinigungsmittel nach Anspruch 3 bis 6, **dadurch gekennzeichnet, dass** die Menge aller Proteasen, bezogen auf die Menge an aktivem Enzymprotein, welche pro Spülgang hinzudosiert wird, bevorzugt 0,001 bis 1000 mg/job, weiter bevorzugt 0,1 bis 600 mg/job und noch weiter bevorzugt 1,0 bis 400 mg/job beträgt.

8. Reinigungsmittel nach einem der vorstehenden Ansprüche, **dadurch gekennzeichnet, dass** das eine Enzym, ggf. zusätzlich zu anderen Enzymen, ausgewählt ist aus der Gruppe der alpha-Amylasen, besonders bevorzugt mindestens eine Amylase ausgewählt aus der Gruppe bestehend aus: die mindestens eine Amylase ausgewählt ist aus:
a) einer α-Amylase, die eine Aminosäuresequenz umfasst, die zu der in SEQ ID NO:5 angegebenen Aminosäuresequenz über deren Gesamtlänge zu mindestens 80% identisch ist und optional mindestens eine Aminosäuresubstitution an einer der Positionen 172, 202, 208, 255 und 261 in der Zählung gemäß SEQ ID NO:5 aufweist, vorzugsweise ausgewählt aus der aus M202L, M202V, M202S, M202T, M202I, M202Q, M202W, S255N, R172Q und Kombinationen davon bestehenden Gruppe; und/oder
b) einer α-Amylase, die eine Aminosäuresequenz umfasst, die zu der in SEQ ID NO:6 angegebenen Aminosäuresequenz über deren Gesamtlänge zu mindestens 60% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine Deletion an einer der Positionen 183 und 184 in der Zählung gemäß SEQ ID NO:6 aufweist, vorzugsweise mindestens eine Aminosäuresubstitution an einer der Positionen 9, 26, 149, 182, 186, 202, 257, 295, 299, 323, 339 and 345, besonders bevorzugt aus der aus R118K, D183*, G184*, N195F, R320K, R458K und Kombinationen davon bestehenden Gruppe ausgewählt; und/oder
c) einer α-Amylase, die eine Aminosäuresequenz umfasst, die zu der in SEQ ID NO:7 angegebenen Aminosäuresequenz über deren Gesamtlänge zu mindestens 90% identisch ist und optional mindestens eine Substitution und/oder Deletion an einer der Positionen 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 in der Zählung gemäß SEQ ID NO:7, aufweist.

9. Reinigungsmittel nach einem der vorstehenden Ansprüche, **dadurch gekennzeichnet, dass** es einen Gelbildner, bevorzugt ausgewählt aus Gelatine, Xanthan und/oder Polyvinylalkohol, insbesondere Gelatine oder Polyvinylalkohol, besonders bevorzugt Polyvinylalkohol, in einer Menge von 4 bis 40, insbesondere von 6 bis 30 Gew.-%, besonders bevorzugt in einer Menge von 7 bis 24 Gew.-%, ganz besonders bevorzugt 8 bis 22 Gew.-%, jeweils bezogen auf das Gesamtgewicht der gelförmigen Phase enthält.

10. Reinigungsmittel nach einem der vorstehenden Ansprüche, **dadurch gekennzeichnet, dass** in der gelförmigen Phase mindestens ein organisches Lösungsmittel enthalten ist, insbesondere ausgewählt aus 1,2-Propandiol, 1,3-Propandiol, Glycerin, 1,1,1-Trimethylolpropan, Triethylenglykol, Dipropylenglykol, Polyethlenglykole und/oder Mischungen daraus.

11. Reinigungsmittel nach Anspruch 10, **dadurch gekennzeichnet, dass** das mindestens ein organisches Lösungsmittel in der gelförmigen Phase in Mengen von 30 bis 90 Gew.-%, insbesondere von 40 bis 85 Gew.-%, besonders bevorzugt von 50 bis 80 Gew.-%, bezogen auf das Gesamtgewicht der gelförmigen Phase, enthalten ist.

12. Reinigungsmittel entsprechend einem der vorstehenden Ansprüche, **dadurch gekennzeichnet, dass** die gelförmige Phase ein Polymer, umfassend sulfonsäuregruppenhaltiges Monomer, insbesondere ein Monomer ausgewählt aus Acrylamidopropansulfonsäuren, Methacrylamidomethylpropansulfonsäuren oder Acrylamidomethylpropansulfonsäure, enthält.

13. Reinigungsmittel nach einem der vorstehenden Ansprüche, **dadurch gekennzeichnet, dass** es zusätzlich mindestens eine feste, insbesondere partikuläre Phase sowie optional mindestens eine weitere flüssige/gelförmige oder feste Phase umfasst.

14. Reinigungsmittel nach einem der vorstehenden Ansprüche, **dadurch gekennzeichnet, dass** es sich um eine Reinigungsmittelportion in einer wasserlöslichen Umhüllung mit einer oder mehreren Kammern/Kompartimenten handelt.

15. Reinigungsmittel nach einem der Ansprüche 1 bis 14, **dadurch gekennzeichnet, dass** die gelförmige Phase in direktem Kontakt, beispielsweise in einer Kammer, mit der mindestens einen festen Phase enthalten ist.

16. Verwendung eines Reinigungsmittels nach einem der Ansprüche 1 bis 15 zur maschinellen Reinigung von Geschirr.
